(19) Europäisches Patentamt
European Patent Office
Office européen des brevets

(11) Numéro de publication: **0 275 762 B1**

(12) **FASCICULE DE BREVET EUROPEEN**

(45) Date de publication de fascicule du brevet: **18.11.93**

(51) Int. Cl.$^5$: **C07D 209/08**, C07D 209/34, C07D 403/12, A61K 31/495, A61K 31/40

(21) Numéro de dépôt: **87402892.1**

(22) Date de dépôt: **17.12.87**

Le dossier contient des informations techniques présentées postérieurement au dépôt de la demande et ne figurant pas dans le présent fascicule.

# (54) Dérivés de l'indole carboxamide ainsi que leurs sels, procédé et intermédiaires de préparation, application à titre de médicaments de ces dérivés et compositions les renfermant.

(30) Priorité: **19.12.86 FR 8617810**

(43) Date de publication de la demande: **27.07.88 Bulletin 88/30**

(45) Mention de la délivrance du brevet: **18.11.93 Bulletin 93/46**

(84) Etats contractants désignés: **AT BE CH DE ES FR GB GR IT LI LU NL SE**

(56) Documents cités:
**EP-A- 0 099 766**
**EP-A- 0 150 139**
**EP-A- 0 201 400**
**FR-A- 2 583 047**

(73) Titulaire: **ROUSSEL-UCLAF**
**35, Boulevard des Invalides**
**F-75007 Paris(FR)**

(72) Inventeur: **Clémence, François**
**2, rue Turgot**
**F-75009 Paris(FR)**
Inventeur: **Guillaume, Jacques**
**62, Rue Pixerecourt**
**F-85020 Paris(FR)**
Inventeur: **Hamon, Gilles**
**40, rue de Bagneux**
**F-92120 Montrouge(FR)**

(74) Mandataire: **Bourgouin, André et al**
**Département des Brevets**
**ROUSSEL UCLAF**
**111, route de Noisy**
**B.P. no 9**
**F-93230 Romainville (FR)**



Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention concerne des dérivés de l'indole carboxamide, ainsi que leurs sels, le procédé et intermédiaires de préparation, l'application à titre de médicaments de ces dérivés, les compositions les renfermant.

La demande européenne EP 0.213.984 décrit des produits dérivés de l'indole carboxamide possédant des propriétés antiarythmiques.

Les demandes européennes EP 0.150.139 et 0.201.400 décrivent des produits possédant également des propriétés antiarythmiques.

Cependant, les produits de ces demandes ont une structure différente de celle des produits de la présente demande.

L'invention a pour objet des dérivés de l'indole carboxamide, ainsi que leurs sels d'addition avec les acides minéraux ou organiques, caractérisés en ce qu'ils répondent à la formule générale (I) :

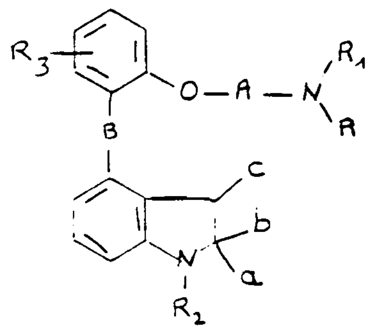

(I)

dans laquelle

- soit $R_2$ et $R_3$ représentent chacun un atome d'hydrogène, B représente une chaîne -CONH-, NH étant du côté de l'indole, b représente un atome d'hydrogène, a et c forment ensemble une seconde liaison entre les carbones qui les portent, A représente une chaîne

  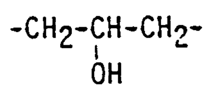

  et ou bien $R_1$ représente un atone d'hydrogène et R représente un radical choisi parmi les radicaux 1,1-diméthylpropyle,

  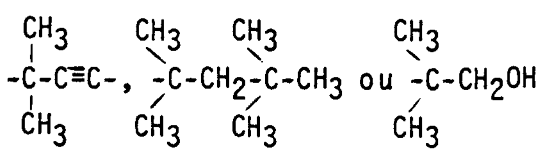

  ou bien R et $R_1$ forment ensemble, avec l'atome d'azote auquel ils sont liés, un noyau morpholine,
- soit $R_2$ et $R_3$ représentent chacun un atome d'hydrogène, B représente une chaîne -CO-NH-, NH étant du côté de l'indole, b représente un atome d'hydrogène et a et c forment ensemble une seconde liaison entre les carbones qui les portent, A représente un chaîne $-(CH_2)_3-$, $R_1$ représente un atome d'hydrogène et R un radical choisi parmi les radicaux 1,1-diméthylpropyle, cyclohexyle, cyclohexylméthyle, propyle, isopropyle ou

  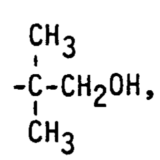

- soit $R_2$ et $R_3$ représentent chacun un atome d'hydrogène, B représente -NH-CO,

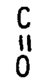

étant du côté de l'indole, b représente un atome d'hydrogène et a et c forment ensemble une seconde liaison entre les carbone qui les portent, A représente la chaîne $-(CH_2)_3-$, $R_1$ représente un atome d'hydrogène et R représente un radical choisi parmi les radicaux cyclopentyle, cyclohexyle, 1,1-diméthylpropyle ou

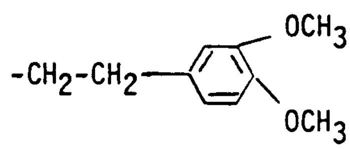

- soit $R_2$ et $R_3$ représentent un atome d'hydrogène, B représente -NH-CO-, -NH étant du côté de l'indole, b représente un atome d'hydrogène et a et c forment ensemble une seconde liaison entre les carbones qui les portent, A représente la chaîne $-(CH_2)_4-$, $R_1$ représente un atome d'hydrogène et R représente le groupement 1,1-diméthyléthyle,
- soit $R_2$ représente un radical méthyle et $R_3$ représente un atome d'hydrogène, B représente -NH-CO, NH étant du côté de l'indole, b représente un atome d'hydrogène et a et c forment ensemble une seconde liaison entre les carbones qui les portent, A représente la chaîne

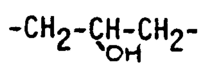

et R représente le groupement 1,1-diméthyléthyle,
- soit les substituants $R_1$ et R forment avec l'atome d'azote auquel ils sont attachés un groupement

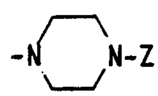

dans lequel Z représente un groupement

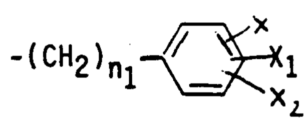

ou un groupement

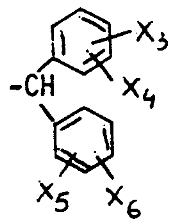

dans lesquels $n_1$ peut prendre les valeurs 1, 2 ou 3 et X, $X_1$, $X_2$, $X_3$, $X_4$, $X_5$ et $X_5$ identiques ou différents représentent un atome d'hydrogène, un radical alkyle renfermant de 1 à 5 atomes de carbone, un radical alkoxy renfermant de 1 à 3 atomes de carbone, un atome d'halogène, un radical

nitro, amino, monoalkylamino ou dialkylamino, à la condition que X, $X_1$ et $X_2$ ne représentent pas tous trois un atone d'hydrogène, A représente une chaîne $-(CH_2)_n-$ dans laquelle n peut prendre les valeurs 2, 3, 4 ou 5 ou une chaîne

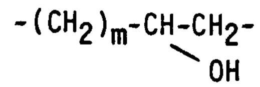

dans laquelle m peut prendre les valeurs 1, 2 ou 3, B représente une chaîne -CO-NH- ou -NH-CO-, $R_3$ représente un atome d'hydrogène, un radical alkyle renfermant de 1 à 5 atomes de carbone, un radical alkoxy renfermant de 1 à 3 atomes de carbone, un atome de chlore, de brome ou d'iode, un radical nitro ou un radical amino éventuellement substitué par un groupe acyle aliphatique renfermant de 2 à 5 atomes de carbone ou par un radical alkyle renfermant de 1 à 5 atomes de carbone, a représente ensemble avec b une fonction oxo, ou représente ensemble avec c une seconde liaison entre les carbone qui les portent, b représente un atome d'hydrogène, ou ensemble avec a une fonction oxo, c représente un atome d'hydrogène, ou ensemble avec a représente une seconde liaison entre les carbones qui les portent, $R_2$ représente un atome d'hydrogène, un radical alkyle linéaire renfermant de 1 à 5 atomes de carbone ou un radical alkyle ramifié renfermant de 3 à 5 atomes de carbone ou un radical alkényle ou alkynyle renfermant de 2 à 5 atomes de carbone ou un radical aralkyle renfermant de 7 à 12 atomes de carbone éventuellement substitué par 1, 2 ou 3 radicaux choisis dans le groupe constitué par les atomes d'halogène et les radicaux méthyle, éthyle, méthoxy, éthoxy, trifluorométhyle, méthylthio, amino et nitro ou un radical cycloalkylalkyle renfermant de 4 à 7 atomes de carbone.

Dans la formule générale (I) et dans ce qui suit, le terme radical alkyle linéaire renfermant de 1 à 5 atomes de carbone désigne, de préférence, un radical méthyle, éthyle ou propyle.

Le terme radical alkyle ramifié renfermant de 3 à 5 atomes de carbone désigne, de préférence, un radical isopropyle ou tert-butyle.

Le terme radical alkoxy désigne un radical méthoxy, éthoxy ou propoxy.

Dans les valeurs alkylamino ou dialkylamino, les radicaux alkyles sont préférentiellement méthyle ou éthyle.

Lorsque X, $X_1$, $X_2$, $X_3$, $X_4$ , $X_5$ et $X_5$ sont des atomes d'halogène, il s'agit de préférence de l'atome de chlore, mais ils peuvent également représenter un atome de fluor, de brome ou d'iode.

Le terme acyle aliphatique renfermant de 2 à 5 atomes de carbone désigne, de préférence, un radical acétyle ou propionyle.

Les termes radical alkényle et alkynyle renfermant de 3 à 5 atomes de carbone désignent, de préférence, un radical allyle ou propargyle.

Le terme radical cycloalkylalkyle renfermant de 4 à 7 atomes de carbone désigne, de préférence, un radical cyclopropylméthyle ou cyclobutylméthyle.

Le terme radical aralkyle renfermant de 7 à 12 atomes de carbone désigne, de préférence, un radical benzyle ou phénéthyle.

Les sels d'addition avec les acides minéraux ou organiques peuvent être, par exemple, les sels formés avec les acides chlorhydrique, bromhydrique, nitrique, sulfurique, phosphorique, acétique, formique, propionique, benzoïque, maléique, fumarique, succinique, tartrique, citrique, oxalique, glyoxylique, aspartique, alcane sulfoniques tels que les acides méthane ou éthane sulfoniques, arylsulfoniques, tels que les acides benzène ou paratoluène sulfoniques et arylcarboxyliques.

Parmi les produits, objet de l'invention, on peut citer notamment les dérivés répondant à la formule (I) ci-dessus, ainsi que leurs sels d'addition avec les acides minéraux ou organiques, caractérisés en ce que, dans ladite formule (I), les substituants $R_1$ et R forment avec l'atome d'azote auquel ils sont liés un groupement

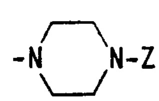

dans lequel Z a la signification précédente, A représente une chaîne

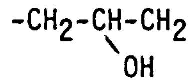

ou $-(CH_2)_3-$ ou $-(CH_2)_4-$, $R_3$ représente un atome d'hydrogène, $R_2$ représente un atome d'hydrogène ou un radical méthyle, b représente un atome d'hydrogène et a et c forment ensemble une seconde liaison entre les carbones qui les portent.

Parmi les produis de l'invention, on peut citer plus particulièrement les produits dont les noms suivent :

- le 2-[3-[(1,1-diméthylpropyl) amino] 2-hydroxypropoxy] N-(1H-indol-4-yl) benzamide et son benzoate et son oxalate neutre,
- le 2-[3-[(1,1-diméthyléthyl) amino] 2-hydroxypropoxy] N-(1-méthyl 1H-indol-4-yl) benzamide et son chlorhydrate,
- le 2-[2-hydroxy 3-[(1,1,3,3-tétraméthylbutyl)amino] propoxy] N-(1H-indol-4-yl) benzamide, et
- le 2-[2-hydroxy 3-[4-(diphénylméthyl) 1-pipérazinyl] propoxy] N-(1H-indol-4-yl) benzamide et son oxalate neutre.

L'invention a également pour objet un procédé de préparation des produits tels que définis par la formule (I) ci-dessus ainsi que de leurs sels, caractérisé en ce que l'on fait réagir un dérivé de formule (II) :

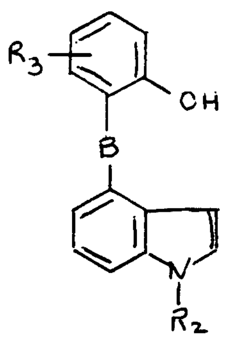

(II)

dans laquelle B, $R_2$ et $R_3$ ont la signification déjà indiquée, avec un halogénure de formule III :

Hal-G (III)

dans laquelle Hal représente un atome de chlore, de brome ou d'iode et -G représente un groupement de formule $-(CH_2)_n-D$, dans laquelle D représente un atome de chlore, de brome, d'iode, ou un radical hydroxy ou un sulfonate de ce radical hydroxy, et n a la signification déjà indiquée, ou -G représente un groupement

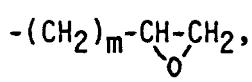

dans laquelle m a la signification déjà indiquée, pour obtenir un dérivé de formule (IV) :

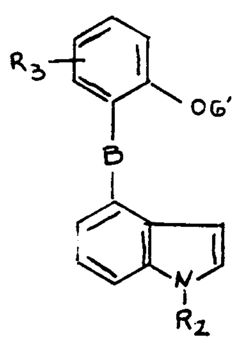

(IV)

dans laquelle B, $R_2$ et $R_3$ ont la signification déjà indiquée, et G' représente un groupement de formule $-(CH_2)_n$-Hal dans laquelle n et Hal ont la signification déjà indiquée, ou un groupement de formule

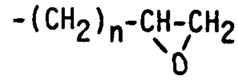

défini ci-dessus, que l'on fait réagir avec une amine de formule (V) :

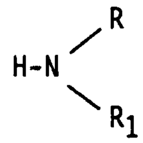

(V)

dans laquelle R et $R_1$ ont la signification déjà indiquée, pour obtenir un produit de formule ($I_A$) :

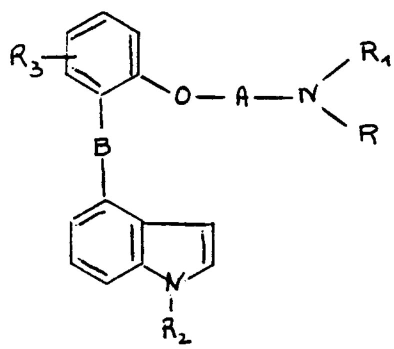

($I_A$)

dans laquelle A, B, R, $R_1$, $R_2$ et $R_3$ ont la signification déjà indiquée, que soit l'on isole et, si désiré, salifie.

Lorsque l'on veut obtenir une chaîne de formule $-(CH_2)-_n$ pour A, si l'on utilise un halogénure de formule III de formule :

Hal-$(CH_2)_n$-D

dans laquelle D représente un atome de chlore, de brome, ou d'iode et Hal a la signification déjà indiquée, il est préférable d'utiliser deux halogènes différents pour éviter la condensation de deux molécules de dérivé de formule IV. C'est ainsi que, par exemple, pour D représentant un atome de chlore, on choisira pour Hal un halogénure plus réactif tel que le bromure.

Lorsque l'on utilise un halogénure hydroxylé de formule (III) répondant à la formule (III') :

HO-$(CH_2)_n$-Hal (III')

dans laquelle n et Hal ont la signification déjà indiquée, pour le faire réagir avec le dérivé de formule (II), on opère alors de préférence en présence de triphényl phosphine et d'azodicarboxylate d'éthyle dans le tétrahydrofuranne.

Avantageusement, on utilise un sulfonate du dérivé de formule (III') ; dans ce cas, de préférence, on utilise comme sulfonate de l'halogénure hydroxylé de formule (III'), son tosylate de formule (III'') :

$T_SO$-$(CH_2)_n$-Hal (III'')

dans laquelle $T_S$ représente un radical tosyle (4-méthylbenzène sulfonate) et n et Hal ont la signification déjà indiquée.

On opère alors par transfert de phase en utilisant comme phase aqueuse de préférence une solution aqueuse d'un hydroxyde alcalin tel que l'hydroxyde de potassium ou de sodium, et comme phase organique non miscible à l'eau un solvant tel que le benzène, en présence d'un agent de transfert tel qu'un sel d'ammonium quaternaire de tétrabutyl ammonium, notamment le bromure ou l'hydrogénosulfate.

La réaction du produit de formule IV avec l'amine de formule V est réalisée par exemple au sein d'un solvant organique inerte tel que le dioxanne, le benzène, le toluène, le diméthylformamide, ou encore un alcool, de préférence l'éthanol, de préférence en présence d'un agent de condensation tel qu'un carbonate ou bicarbonate alcalin comme le carbonate de potassium, un hydroxyde alcalin comme la soude ou la potasse, ou une amine tertiaire comme la triéthylamine. On peut opérer également en utilisant comme solvant directement l'amine de formule (V).

Lorsque l'on veut obtenir une chaîne de formule

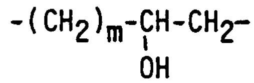

pour A, on utilise un halogénure de formule Hal

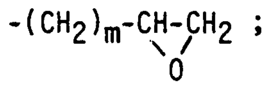

dans ce cas, Hal représente de préférence un atome de chlore. La réaction du dérivé de formule (II) avec l'halogénure de formule (III) est alors réalisée de préférence en présence d'une base telle que le carbonate de potassium ou de sodium, la soude ou la potasse.

La réaction du dérivé de formule (IV) dans laquelle C représente une chaîne

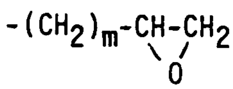

avec l'amine de formule (V) est réalisée soit directement en utilisant l'amine comme solvant, soit en utilisant un solvant tel qu'un alcool aliphatique comme l'éthanol.

Dans une variante du procédé décrit ci-dessus pour la préparation des dérivés de formule (I), dans laquelle A représente une chaîne $-(CH_2)_n-$, on fait réagir un dérive de formule II avec un dérivé de formule (VI) :

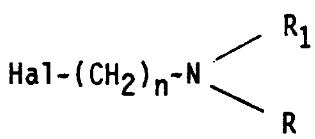

(VI)

dans laquelle Hal, n, R et $R_1$ ont la signification déjà indiquée, pour obtenir le dérivé de formule $I_A$ recherché que l'on isole et si désiré salifie.

On fait réagir le dérivé de formule (VI) sous forme d'amine libre ou, de préférence sous forme de sel comme un chlorhydrate.

L'invention a également pour objet un procédé de préparation des dérivés de formule (I) dans laquelle les substituants $R_1$ et R forment avec l'atome d'azote auquel ils sont attachés un groupement

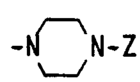

dans lequel Z a la signification déjà indiquée, caractérisé en ce que l'on fait réagir un produit de formule (IV) :

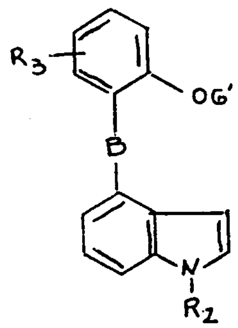

(IV)

dans laquelle B, $R_2$, $R_3$ et G' ont la signification déjà indiquée avec une amine de formule (V') :

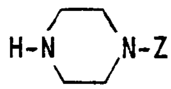

(V')

dans laquelle Z a la signification déjà indiquée pour obtenir un produit de formule ($I_B$) :

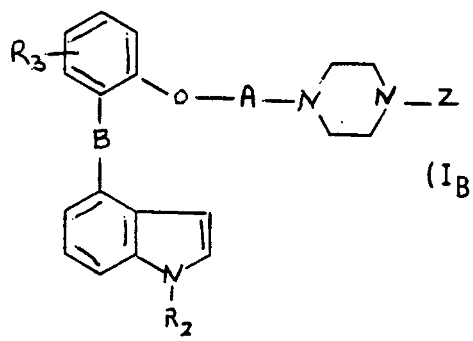

($I_B$)

dans laquelle A, B, $R_2$, $R_3$ et Z ont les significations déjà indiquées, que soit l'on isole, et si désiré salifie, soit si désiré, soumet à l'action d'un agent d'halogénation pour obtenir un produit de formule (VII) :

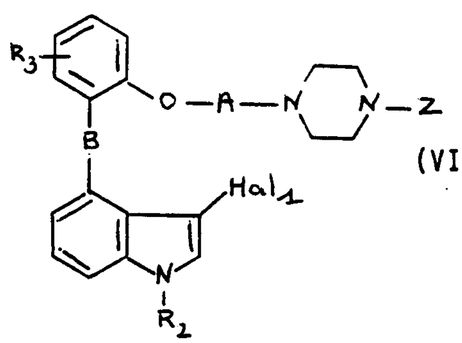

(VII)

dans laquelle $Hal_1$ représente un atome de brome ou de chlore, que l'on soumet à une hydrolyse pour obtenir un composé de formule ($I_C$) :

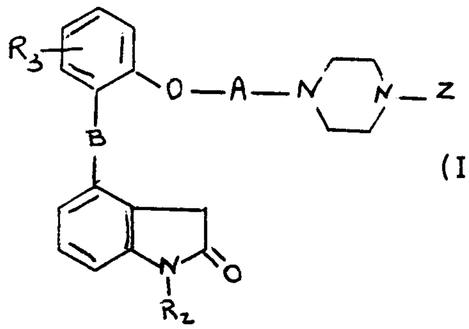

$(I_C)$

dans laquelle A, B, $R_2$, $R_3$ et Z ont les significations précédentes, que l'on isole et si désiré salifie par action d'un acide minéral ou organique.

Dans une variante du procédé ci-dessus décrit pour la préparation des dérivés de formule (I) dans laquelle A représente une chaîne $-(CH_2)_n-$l'on fait réagir un produit de formule (II) :

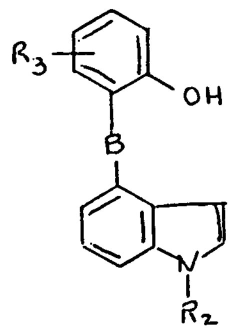

(II)

dans laquelle B, $R_2$ et $R_3$ ont les significations précédentes avec un composé de formule (VIII) :

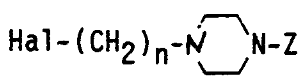

(VIII)

dans laquelle Hal représente un atome de chlore, de brome ou d'iode et n et Z ont les significations précédentes, pour obtenir un composé de formule ($I_B$) correspondant que l'on transforme si désiré en composé de formule ($I_C$) correspondant.

La réaction du produit de formule (IV) avec l'amine de formule (V') est réalisée selon des conditions identiques à celles indiquées pour la réaction des produits de formule (IV) et (V).

La réaction du produit de formule (II) avec le composé de formule (VIII) est réalisée selon les conditions indiquées pour la réaction des produits (II) et (VI).

L'halogénation des dérivés de formules $I_A$ peut être utilisée, par exemple, à l'aide du complexe bromé de la pyridine de formule :

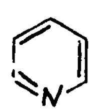

, $Br_2$ , HBr

dans le cas de la bromation. Elle est réalisée avantageusement à l'aide d'un N-halo succinimide, de préférence le N-bromo ou le N-chloro succinimide : on opère dans le dioxanne ou de préférence dans l'acide acétique. Le produit de formule (VII) obtenu est de préférence un produit chloré.

L'hydrolyse du produit de formule (VII) est réalisée, de préférence à l'aide d'un acide minéral tel que l'acide phosphorique, l'acide sulfurique, ou de préférence l'acide chlorhydrique en solution aqueuse. Cette solution peut être utilisée concentrée, mais de préférence diluée par exemple en solution normale. On peut utiliser, en outre, un solvent tel qu'un alcool aliphatique comme l'éthanol.

Les produits de formule (II) utilisés au départ des procédés ci-dessus décrits sont des produits connus ou peuvent être préparés comme indiqué dans la demande de brevet européen 0 213 984.

Les produits de formule (VII) telle que définie ci-dessus sont des produits de formule (VII) à titre de produits industriels nouveaux.

Les dérivés objet de la présente demande possèdent de très intéressantes propriétés pharmacologiques ; ils sont doués, notamment de certaines propriétés antiarythmiques. De plus, certains dérivés, objet de la présente demande, possèdent par ailleurs des propriétés anticalciques et bétabloquantes, hypotensives et vasodilatatrices.

Ces propriétés sont illustrées plus loin dans la partie expérimentale.

Ces propriétés justifient l'utilisation des nouveaux dérivés du benzamide et de leurs sels à titre de médicaments.

La présente demande a ainsi, également pour objet l'application, à titre de médicaments, des nouveaux dérivés du benzamide, tels que définis ci-dessus, ainsi que de leurs sels d'addition avec les acides pharmaceutiquement acceptables.

Parmi les médicaments préférés de l'invention, on retient, tout particulièrement les dérivés répondant à la formule (I) ci-dessus, ainsique leurs sels d'addition avec les acides minéraux ou organiques pharmaceutiquement acceptables, caractérisés en ce que, dans ladite formule (I), les substituants $R_1$ et R forment avec l'atome d'azote auquel ils sont liés un groupement

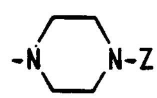

dans lequel Z a la signification précédente, A représente une chaîne

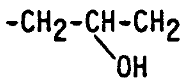

ou $-(CH_2)_3-$ ou $-(CH_2)_4-$, $R_3$ représente un atome d'hydrogène, $R_2$ représente un atome d'hydrogène ou un radical méthyle, b représente un atome d'hydrogène et a et c forment ensemble une seconde liaison entre les carbones qui les portent.

Parmi les médicaments de l'invention, on peut citer plus particulièrement les produits dont les noms suivent :

- le 2-[3-[(1,1-diméthylpropyl) amino] 2-hydroxypropoxy] N-(1H-indol-4-yl) benzamide et son benzoate et son oxalate neutre,
- le 2-[3-[(1,1-diméthyléthyl) amino] 2-hydroxypropoxy] N-(1-méthyl 1H-indol-4-yl) benzamide et son chlorhydrate,
- le 2-[2-hydroxy 3-[(1,1,3,3-tétraméthylbutylamino] propoxy] N-(1H-indol-4-yl) benzamide, et
- le 2-[2-hydroxy 3-[4-(diphénylméthyl) 1-pipérazinyl] propoxy] N-(1H-indol-4-yl) benzamide et son oxalate neutre.

Les médicaments, objet de la présente invention, trouvent, par exemple, leur emploi dans le traitement des arythmies et de l'angor.

La dose usuelle, variable selon le dérivé utilisé, le sujet traité et l'affection en cause, peut être, par exemple, de 50 mg à 1g par jour. Par voie orale chez l'homme, le dérivé de l'exemple 1 peut être administré à la dose quotidienne de 200 mg à 800 mg, par exemple, pour le traitement des arythmies venticulaires, supraventiculaires et jonctionnelles, soit environ de 3 mg à 12 mg par kilogramme de poids corporel.

L'invention a enfin pour objet les compositions pharmaceutiques qui renferment, au moins, un dérivé précité ou l'un de ses sels d'addition avec les acides pharmaceutiquement acceptables, à titre de principe actif.

A titre de médicaments, les dérivés, objet de la présente demande, et leurs sels d'addition avec les acides pharmaceutiquement acceptables peuvent être incorporés dans des compositions pharmaceutiques destinées à la voie digestive ou parentérale.

Ces compositions pharmaceutiques peuvent être, par exemple, solides ou liquides et se présenter sous les formes pharmaceutiques couramment utilisées en médecine humaine, comme par exemple, les

comprimés, simples ou dragéifiés, les gélules, les capsules, les granulés, les suppositoires, les préparations injectables ; elles sont préparées selon les méthodes usuelles. Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycoles, les divers agents mouillants, dispersants ou émulsifiants, les conservateurs.

Il va être donné maintenant, à titre non limitatif, des exemples de mise en oeuvre de l'invention.

**Préparation du 2-hydroxy N-(1H-indol 4-yl) benzamide utilisé au départ l'exemple n° 1.**

On ajoute lentement sous agitation et atmosphère inerte 92 cm3 d'une solution de triisobutylaluminium en solution à 1,1 mole/l dans le toluène, à une solution de 6,6 g de 4-amino indole dans 250 cm3 de chloroforme, ajoute ensuite 9,6 cm3 de salicylate de méthyle, porte au reflux pendant 20 heures, refroidit à température amibiante, ajoute 300 cm3 d'acide chlorhydrique N et 300 cm3 de chlorure de méthylène, lave à l'eau, sèche, amène à sec sous pression réduite à 50 °C, empâte à l'éther, filtre, sèche à 60 °C sous pression réduite et obtient 9,4 g du produit attendu F° ≃ 232 °C.

Spectre UV-(Ethanol) :

| | | |
|---|---|---|
| infl. 216 nm | $E_1^1$ = 1 595 | |
| infl. 233 nm | $E_1^1$ = 680 | $\epsilon$ = 17 200 |
| infl. 262 nm | $E_1^1$ = 187 | |
| infl. 303 nm | $E_1^1$ = 482 | $\epsilon$ = 12 200 |
| infl. 314 nm | $E_1^1$ = 494 | $\epsilon$ = 12 500 |

**Exemple 1 : 2-[3-[(1,1-diméthylpropyl) amino] 2-hydroxy propoxy] N-(1H-indol 4-yl) benzamide et son oxalate neutre.**

**Stade A :** 2-[(2-oxiranyl) méthoxy] N-(1H-indol 4-yl) benzamide

On chauffe au reflux pendant 30 heures, sous atmosphère inerte, une solution de 3,5 g de 2-hydroxy N-(1H-inol 4-yl) benzamide et 1,9 g de carbonate de potassium dans 100 cm3 d'acétone avec 11 cm3 de d'épichlorhydrine, filtre l'insoluble, amène à sec le filtrat, purifie le résidu par chromatographie sur silice (éluant : chloroforme-acétone, TEA 6-3-1) amène à sec les fractions de Rf 0,45, empâte à l'éther, filtre, sèche sous pression réduite à 60 °C et obtient 3,65 g du produit attendu F° = 171 °C.

**Stade B :** 2-[3-[(1,1-diméthylpropyl) amino] 2-hydroxy propoxy] **N-(1H-indol 4-yl) benzamide et son oxalate neutre.**

Base :

On chauffe au reflux sous agitation et atmosphère inerte pendant 5 heures 3,5 g de produit du stade A en solution dans 35 cm3 d'éthanol avec 3 cm3 de terpentylamine, évapore le solvant, purifie par chromatographie sur silice (éluant chlorure de méthylène-méthanol 9-1) et obtient 3,5 g du produit attendu.

Formation de l'oxalate neutre :

On dissout 2,4 g de base ci-dessus dans 20 cm3 d'éthanol, ajoute 0,245 g d'acide oxalique déhydraté, filtre le sel formé, sèche, recristallise dans l'éthanol et obtient 1,83 g du produit attendu F° ≃ 180 °C.

**Exemple 2 : 2-[3-(4-morpholinyl) 2-hydroxy propoxy] N-(1H-indol 4-yl) benzamide et son chlorhydrate**

On opère comme indiqué au stade B de l'exemple 1 à partir de 4 g de produit au stade A de l'exemple 1 et 1,7 cm3 de morpholine. On obtient 4,12 g de produit attendu après chromatographie sur silice (éluant

chloroforme - acétate d'éthyle - triéthylamine 6-3-1).

On prépare le chlorhydrate avec une solution de chlorure de méthylène saturée en acide chlorhydrique.

Fusion du chlorhydrate après recristallisation dans l'éthanol = 210°C.

**Exemple 3 : 2-[3-[(1,1-diméthylpropyl) amino] propoxy] N-(1H-indol 4-yl) benzamide et son chlorhydrate**

**Stade A :** 2-(3-chloropropoxy) N-(1H-indol 4-yl) benzamide.

On prépare sous agitation et atmosphère inerte une solution de 5 g de 2-hydroxy N-(1H-indol 4-yl) benzamide, de 400 cm3 de tétrahydrofuranne, de 1,8 cm3 3-chloropropanol et de 5,7 g de triphénylphosphine, ajoute lentement 3,4 cm3 d'azodicarboxylate d'éthyle, laisse pendant 5 heures sous agitation, ajoute 5,7 g de triphénylphosphine, 1,8 cm3 de 3-chloropropanol puis, lentement, 3,4 cm3 d'azodicarboxylate d'éthyle, laisse à nouveau sous agitation pendant 15 heures, amène à sec, purifie par chromatographie sur silice (Eluant : benzène - acétate d'éthyle : 95-5), amène à sec les fractions de Rf 0,15, empâte à l'éther, filtre, sèche sous pression réduite et obtient 5,5 g du produit attendu. F°≃ 140°C.

**Stade B :** 2-[3-[(1,1-diméthylpropyl) amino] propoxy] N-(1H-indol 4-yl benzamide et son chlorhydrate.

Base :

On chauffe à 120°C pendant 5 heures 2 g de produit du stade A dans 20 cm3 d'éthanol avec 2 cm3 de terpentylamine et en présence de 0,84 g de carbonate de potassium, filtre, évapore le solvant, purifie par chromatographie sur silice (Eluant : chlorure de méthylène - méthanol 9-1) et obtient 2 g du produit attendu.

Chlorhydrate :

On dissout 1,45 g de base dans 20 cm3 d'ispropanol à 50°C , ajoute jusqu'à pH acide une solution d'acétate d'éthyle saturé en acide chlorhydrique, glace, filtre, sèche, recristallise dans l'isopropanol et obtient 1,7 g du produit attendu. F°≃ 216°C.

**Exemple 4 : 2-[3-(cyclohexylamino) propoxy] N-(1H-indol-4-yl) benzamide et son chlorhydrate.**

On opère comme indiqué à l'exemple 3, mais en remplaçant la terpentylamine par la cyclohexylamine et chromatographie sur silice éluant ($CHCl_3$-acétate d'éthyle, TEA: 6-31), obtient 2,1 g de produit attendu F°≃ 148°C (base), puis forme le chlorhydrate qui fond à 214°C après recristallisation dans l'isopropanol.

**Exemple 5 : 2-[3-(cyclohexylméthyl) amino] propoxy N-(1H-indol-4-yl) benzamide et son chlorhydrate.**

On opère comme indiqué à l'exemple 3, mais en remplaçant la terpentylamine par la cyclohexyl méthylamine et obtient le produit attendu, puis son chlorhydrate que l'on recristallise dans l'éthanol. F°≃ 228°C.

**Exemple 6 : 2-[4-(1,1-diméthyléthyl) amino] butoxy] N-[1H-indol-4-yl) benzamide et son fumarate acide.**

On opère comme à l'exemple 3, mais à 50°C et en remplaçant le terpentylamine par la terbutylamine, et le 2-(3-chloropropoxy) N-1H-indol 4-yl benzamide par le 2-(4-bromobutoxy) N-(1H-indol 4-yl) benzamide, chromatographie sur silice éluant ($CHCl_3$, acétone, TEA 6-31), reprend à l'éther pour obtenir le produit attendu F°≃ 147°C (base). On prépare le fumarate acide qui fond à 224-225°C avec sublimation, après recristallisation dans l'éthanol.

**Préparation du 2-(4-bromobutoxy) N-(1H-indol-4-yl) benzamide**

On porte au reflux pendant 1 H 15 mn, une suspension de 7,5 g de 2-hydroxy N-(1H-indol 4-yl) benzamide et de 8,28 g de carbonate de potassium dans 150 cm3 d'acétone, avec 18 cm3 de 1,4

dibromobutane,laisse refroidir, filtre le précipité, rince à l'acétone, amène à sec le filtrat, purifie par chromatographie sur silice (Eluant : chlorure de méthylène - acétate d'éthyle 9-1), puis recristallisation dans l'acétate d'éthyle et obtient le produit attendu ; F° ≃ 135°C.

**Exemple 7 : 2-[3-[(1,1-diméthyléthyl) amino] 2-hydroxy propoxy] N-(1-méthyl 1H-indol-4-yl) benzamide et son chlorhydrate**

**Stade A :** 2-[(2-oxiranyl) méthoxy] N-(1-méthyl 1H-indol-4-yl) benzamide.

On opère comme indiqué à l'exemple 1, stade A, mais à partir du 2-hydroxy N-(1-méthyl) 1H-indol 4-yl) benzamide pour obtenir le produit attendu F° ≃ 160°C.

**Stade B :**

On opère comme indiqué à l'exemple 1, stade B, mais à partir du produit obtenu ci-dessus en remplaçant la terpentylamine par la terbutylamine pour obtenir le produit attendu F° ≃ 130°C (base). On prépare le chlorhydrate qui fond à 195°C après recristallisation dans l'isopropanol.

**Préparation de la 2-hydroxy N-(1-méthyl 1H-indol-4-yl) benzamide de départ de l'exemple 7**

On porte au reflux pendant 24 heures une solution de 5 g de 1-méthyl 1H-indol-4-amine préparée selon Steven V. Ley (J. Chem Soc. Chem. Com 1982 p. 1356), de 4,7 g d'acide salicylique et de 7 g de dicyclohexylcarbodiimide dans 80 cm3 de tétrahydrofuranne, rajoute au bout de 5 heures de reflux 20 % d'acid salicylique et de dicyclohexyl carbodiimide, filtre, évapore le filtrat sous pression réduite, purifie le résidu par chromatographie sur silice (Eluant : chlorure de méthylène), et obtient 5,5 g du produit attendu F° ≃ 211°C.

**Exemple 8 : 2-[3-[(1,1-diméthyl 2-propynyl) amino] 2-hydroxy propoxy] N-(1H-indol-4-yl) benzamide et son oxalate neutre.**

On opère comme au stade B de l'exemple 1 à partir de 4 g du produit préparé comme au stade A de l'exemple 1 et 1,5 cm3 de 1,1-diméthylpropargyl amine. On obtient 3,77 g de base attendue après chromatographie sur silice (éluant : chloroforme-acétate d'éthyle-triéthylamine 6-3-1).

Oxalate neutre.

On dissout 3,09 g de base dans 100 cm3 d'éthanol et ajoute 0,497 g d'acide oxalique. On glace, filtre, sèche sous pression réduite à 80°C, recristallise dans l'éthanol et obtient 2,5 g d'oxalate neutre attendu. F ≃ 160°C.

| Analyse : $C_{23}H_{25}N_3O_3$ = 872,984 | | | | | | |
|---|---|---|---|---|---|---|
| Calculé | C% | 66,04 | H% | 6,00 | N% | 9,63 |
| Trouvé | C% | 65,9 | H% | 6,0 | N% | 9,5 |

**Exemple 9 : 2-[2-hydroxy 3-[(1,1,3,3-tétraméthylbutyl) amino] propoxy] N-(1H-indol-4-yl) benzamide.**

On opère comme à l'exemple 1, stade B, à partir de 5 g du produit préparé comme au stade A de l'exemple 1 et 4,9 cm3 de ter-octylamine. On obtient 4,27 g de produit attendu. F = 140°C.

| Analyse : $C_{26}H_{35}N_3O_3$ = 437,587 | | | | | | |
|---|---|---|---|---|---|---|
| Calculé | C% | 71,37 | H% | 8,06 | N% | 9,60 |
| Trouvé | C% | 71,6 | H% | 8,3 | N% | 9,5 |

**Exemple 10 : 2-[3-[(1,1-diméthyl 2-hydroxyéthyl) amino] 2-hydroxy propoxy] N-(1H-indol-4-yl) benzamide et son oxalate neutre.**

On opère comme à l'exemple 1, stade B, à partir de 4 g du produit préparé comme au stade A de l'exemple 1 et 1,3 cm3 de 2-amino 2-méthyl 1-propanol et obtient 3,33 g de base attendue que l'on transforme en oxalate neutre comme indiqué à l'exemple 8. On obtient 2,8 g d'oxalate attendu. F ≃ 180 ° C.

| Analyse : $C_{22}H_{27}N_3O_4$ = 884,99 | | | | | | |
|---|---|---|---|---|---|---|
| Calculé | C% | 62,43 | H% | 6,38 | N% | 9,50 |
| Trouvé | C% | 62,3 | H% | 6,6 | N% | 9,4 |

**Exemple 11 : N-(1H-indol-4-yl) 2-[3-(propylamino) propoxy] benzamide et son chlorhydrate.**

On opère comme au stade B de l'exemple 3 à partir de 4,5 g de produit préparé comme au stade A de l'exemple 3 et 6,5 cm3 de N-propylamine. On obtient après chromatographie sur silice (éluant : chloroforme-acétate d'éthyle-triéthylamine 6-3-1) 3,20 g de base attendue. 3 g de cette base sont transformés en chlorhydrate comme indiqué à l'exemple 3. On récupère 2 g de chlorhydrate attendu. F = 202 ° C.

| Analyse : $C_{21}H_{25}N_3O_2$ = 387,913 | | | | | | | | |
|---|---|---|---|---|---|---|---|---|
| Calculé | C% | 65,02 | H% | 6,76 | Cl% | 9,14 | N% | 10,83 |
| Trouvé | C% | 64,9 | H% | 6,7 | Cl% | 9,3 | N% | 10,7 |

**Exemple 12 : N-(1H-indol-4-yl) 2-[3-(1-méthyléthyl) amino] propoxy] benzamide et son chlorhydrate.**

On opère comme à l'exemple 11 à partir de 4 g de produit de départ et 10 cm3 d'isopropylamine. Onobtient 2,93 g de base attendue et 1,79 g de chlorhydrate attendu à partir de 2,6 g de base. Chlorhydrate : F = 180 ° C.

| Analyse : $C_{21}H_{25}N_3O_2$, HCl = 387,913 | | | | | | | | |
|---|---|---|---|---|---|---|---|---|
| Calculé | C% | 65,02 | H% | 6,76 | Cl% | 9,14 | N% | 10,83 |
| Trouvé | C% | 65,0 | H% | 6,9 | Cl% | 9,0 | N% | 10,8 |

**Exemple 13 : 2-[3-[(1,1-diméthyl 2-hdyroxyéthyl) amino] propoxy] N-(1H-indol-4-yl) benzamide et son fumarate neutre.**

On opère comme indiqué à l'exemple 3, stade B à partir de 3 g de produit préparé comme au stade A de l'exemple 3 et 5 cm3 de 2-amino 2-méthyl 1-propanol. Après chromatographie sur silice (éluant : chloroforme-méthanol-triéthylamine 90-05-05), on obtient 2,1 g de base.

Fumarate neutre.

En utilisant 1,85 g de base et 5,63 g d'acide fumarique, on obtient 1,323 g de fumarate neutre attendu. F = 186 ° C.

| Analyse : $(C_{22}H_{27}N_3O_3)_3$ $C_4H_4O_4$ = 879,031 | | | | | | |
|---|---|---|---|---|---|---|
| Calculé | C% | 65,59 | H% | 6,65 | N% | 9,56 |
| Trouvé | C% | 65,5 | H% | 6,9 | N% | 9,3 |

**Exemple 14 : N-[2-[3-[(1-méthyléthyl) amino] propoxy] phényl] 1H-indol-4-carboxamide et son chlorhydrate.**

**Stade A :** N-[2-(3-bromopropoxy) phényl] 1H-indol-4-carboxamide.

On met en suspension comprenant 2 g de N-(2-hydroxyphényl) 1H-indol-4-carboxamide et 2,18 g de carbonate de potassium dans 20 cm3 d'acétone, ajoute 3,2 cm3 de 1,3-dibromopropane et porte au reflux pendant 1 heure et demie, filtre, élimine le solvant sous pression réduite, chromatographie le résidu sur silice (éluant : chloroforme-acétat d'éthyle-triéthylamine 6-3-1) et obtient 2,37 g de produit attendu. F = 145 °C.

**Stade B :** N-[2-[3-[(1-méthyléthyl) amino] propoxy] phényl] 1H-indol 4-carboxamide et son chlorhydrate.

On opère comme au stade B de l'exemple 3 à partir de 4 g de produit préparé au stade A précédent et 4,5 cm3 d'isopropylamine. Après chromatographie sur silice (éluant : chloroforme-acétate d'éthyle-triéthylamine 6-3-1), on obtient 2,91 g de base puis 2,5 g de chlorhydrate. F = 234 °C.

| Analyse : $C_{21}H_{25}N_3O_2$, HCl = 387,913 | | | | | | | | |
|---|---|---|---|---|---|---|---|---|
| Caclulé | C% | 65,02 | H% | 6,76 | Cl% | 9,14 | N% | 10,83 |
| Trouvé | C% | 65,3 | H% | 6,8 | Cl% | 9,3 | N% | 10,7 |

**Exemple 15 : N-[2-[3-(cyclopentylamino) propoxy] phényl] 1H-indol-4-carbonxamide et son chlorhydrate.**

On opère comme à l'exemple 14, stade B, à partir de 4 g de produit préparé au stade A et 2,11 cm3 de cyclopentylamine et obtient 3,3 g de base attendue puis 2,8 g de chlorhydrate. F = 244 °C.

| Analyse : $C_{21}H_{27}N_3O_2$, HCl = 413,95 | | | | | | | | |
|---|---|---|---|---|---|---|---|---|
| Calculé | C% | 66,74 | H% | 6,82 | Cl% | 8,56 | N% | 10,15 |
| Trouvé | C% | 66,9 | H% | 6,8 | Cl% | 8,4 | N% | 9,9 |

**Exemple 16 : N-[2-[3-(cyclohexylamino) propoxy] phényl] 1H-indol-4-carboxamide et son chlorhydrate.**

On opère comme à l'exemple 14, stade B à partir de 2,5 g de produit de départ et 1,5 cm3 de cyclohexylamine, on obtient 1,8 g de base attendue et 1,7 g de chlorhydrate attendu. F 260 °C.

| Analyse : $C_{24}H_{29}N_3O_2$, HCl = 427,979 | | | | | | | | |
|---|---|---|---|---|---|---|---|---|
| Calculé | C% | 67,35 | H% | 7,06 | Cl% | 8,28 | N% | 9,82 |
| Trouvé | C% | 67,1 | H% | 7,1 | Cl% | 8,4 | N% | 9,7 |

**Exemple 17 : N-[2-[3-[(1,1-diméthylpropyl) amino] propoxy] phényl] 1H-indol-4-carboxamide et son chlorhydrate.**

On opère comme à l'exemple 14, stade B, à partir de 4 g du produit de départ et 5 cm3 de teramylamine. On obtient 2,90 g de base attendue puis 2,5 g de chlorhydrate attendu à partir de 2,7 g de base. F = 230 °C.

| Analyse : $C_{23}H_{29}N_3O_2$, HCl = 415,967 | | | | | | | | |
|---|---|---|---|---|---|---|---|---|
| Calculé<br>Trouvé | C%<br>C% | 66,41<br>66,4 | H%<br>H% | 7,27<br>7,3 | Cl%<br>Cl% | 8,52<br>8,6 | N%<br>N% | 10,1<br>10,1 |

**Exemple 18 : N-[2-[3-[2-(3,4-diméthoxyphényl) éthyl] amino] propoxy] phényl] 1H-indol-4-carboxamide et son chlorhydrate.**

On opère comme à l'exemple 14, stade B à partir de 3 g du produit de départ et 2,31 cm3 d'homovératrylamine et obtient après chromatograhie sur silice (éluant : chloroforme-méthanol 9-1) 2,75 g de base attendu puis 1,75 g de chlorhydrate à partir de 2,55 g de base. F = 182 °C.

| Analyse : $C_{28}H_{31}N_3O_4$, HCl = 510,038 | | | | | | | | |
|---|---|---|---|---|---|---|---|---|
| Calculé<br>Trouvé | C%<br>C% | 65,94<br>65,9 | H%<br>H% | 6,32<br>6,2 | Cl%<br>Cl% | 6,95<br>6,7 | N%<br>N% | 8,24<br>8,2 |

**Exemple 19 : 2-[2-hydroxy 3-[4-(diphénylméthyl) 1-pipérazinyl] propoxy] N-(1H-indol-4-yl) benzamide et son oxalate neutre.**

On opère comme à l'exemple 1, stade B à partir de 3,08 g de 2-[(2-oxiranyl) méthoxy] N-(1H-indol-4-yl) benzamide préparé comme au stade A de l'exemple 1 et 5,2 g de 1-(diphénylméthyl) pipérazine. On obtient après chromatographie sur silice (éluant : chloroforme-acétate d'éthyle 7-3) 5,12 g de base attendue puis 3,22 g d'oxalate neutre attendu à partir de 4,36 g de base. F ≃ 170 °C.

| Analyse : $(C_{35}H_{36}N_4O_3)_2$ $C_2H_2O_4$ = 1211,44 | | | | | | |
|---|---|---|---|---|---|---|
| Calculé<br>Trouvé | C%<br>C% | 71,38<br>71,4 | H%<br>H% | 6,15<br>6,3 | N%<br>N% | 9,24<br>9,1 |

**Exemple 20 : N-(1H-indol-4-yl) 2-[4-[4-[2-(3,4,5-triméthoxyphényl) éthyl] 1-pipérazinyl] butoxy] benzamide et son difumarate.**

On chauffe à 60 °C pendant 10 heures 2,5 g de 2-(4-bromobutoxy) N-(1H-indol-4-yl) benzamide préparé comme à l'exemple 6, 2,71 g de 1-[2-(3,4,5-triméthoxyphényl) éthyl] pipérazine (Hoechst AG DE 3 347 173), 0,683 g de carbonate de sodium dans 25 cm3 d'éthanol. On refroidit, verse dans l'eau, extrait à l'acétate d'éthyle, lave à l'eau, sèche et élimine les solvants sous pression réduite. On chromatographie le résidu sur silice (éluant : chloroforme-acétone-triéthylamine 6-3-1) et récupère 3,51 g de base attendue.

Difumarate.

En utilisant 2,52 g de base et 498 mg d'acide fumarique, on obtient 1,718 g de difumarate attendu. F = 182-183 °C.

| Analyse : $(C_{34}H_{42}N_4O_5)_2$ $C_8H_8O_8$ = 818,895 | | | | | | |
|---|---|---|---|---|---|---|
| Calculé<br>Trouvé | C%<br>C% | 61,60<br>61,3 | H%<br>H% | 6,15<br>6,1 | N%<br>N% | 6,84<br>6,6 |

**Exemple 21 : Benzoate de 2-[3-[(1,1-diméthylpropyl) amino] 2-hydroxy propoxy] N-(1H-indol-4-yl) benzamide.**

On ajoute 1,11 g d'acide benzoïque dans une solution de 3,6 g de base obtenue au stade B de l'exemple 1. On glace, filtre, sèche sous pression réduite à 90 °C et obtient 3 g de produit attendu après recristallisation dans l'isopropanol. F = 179 °C.

| Analyse : $C_{23}H_{29}N_3O_3$ = 517,615 | | | | | | |
|---|---|---|---|---|---|---|
| Calculé | C% | 69,61 | H% | 6,82 | N% | 8,12 |
| Trouvé | C% | 69,9 | H% | 6,8 | N% | 8,2 |

**Exemple 22 : 2-[2-hydroxy 3-[4-(diphénylméthyl) 1-pipérazinyl] propoxy] N-(1-méthyl 1H-indol-4-yl) benzamide et son chlorhydrate.**

On opère comme à l'exemple 1, stade B en utilisant au départ 4 g de 2-[(2-oxiranyl) méthoxy] N-(1-méthyl 1H-indol-4-yl) benzamide préparé à l'exemple 7 et 6,3 g de diphénylméthyl pipérazine. On obtient 5,95 g de base puis 4,9 g de chlorhydrate attendu. F = 196 °C après recristallisation dans l'éthanol.

| Analyse : $C_{36}H_{38}N_4O_3$, HCl = 611,19 | | | | | | | | |
|---|---|---|---|---|---|---|---|---|
| Calculé | C% | 70,76 | H% | 6,43 | Cl% | 5,80 | N% | 9,17 |
| Trouvé | C% | 70,4 | H% | 6,3 | Cl% | 5,9 | N% | 9,1 |

On prépare aussi de manière analogue à celle déjà décrite dans les exemples le produit suivant :

- le 2-[2-hydroxy 3-[[4-bis (4-fluorophényl) méthyl] 1-pipérazinyl] propoxyl] phényl] N-(1H-indol-4-yl) benzamide.

**Exemple 23 :**

On a préparé des comprimés répondant à la formule :

| | |
|---|---|
| - oxalate neutre de 2-[3-[(1,1-diméthylpropyl) amino] 2-hydroxy propoxy] N-(1H-indol-4-yl) benzamide | 50 mg ; |
| - excipient q. s. pour un comprimé terminé à | 100 mg. |
| (Détail de l'excipient : lactose, amidon, talc, stéarate de magnésium). | |

**Exemple 24 :**

On a préparé des comprimés répondant à la formule :

| | |
|---|---|
| - chlorhydrate de 2-[3-[(1,1-diméthyléthyl) amino] 2-hydyroxy propoxy] N-(1-méthyl 1H-indol-4-yl) benzamide | 100 mg. |
| - Excipient q.s. pour un comprimé terminé à | 150 mg. |
| (Détail de l'excipient : lactose, amidon, talc, stéarate de magnésium). | |

**ETUDE PHARMACOLOGIQUE**

1) Affinité pour les récepteurs béta$_1$ adrénergiques

La technique est inspirée de celle de Möhler et Okada : Science, Vol. 198 p. 849-851 (1977).

On homogénéise dans 90 ml de sucrose 0,32 M, 10 cortex prélevés sur des cerveaux de rats mâles pesant 150 g en moyenne. Après centrifugation à 1000 g du mélange homogénéisé pendant 20 minutes à 0 °C, le surnageant est centrifugé à 30 000 g pendant 15 minutes à 0, +4 °C.

Le culot est mis en suspension dans 120 ml de tampon Tris HCl 50mM pH 7,7 et centrifugé à 30 000 g pendant 15 minutes à 0, + 4 °C. Le nouveau culot obtenu est mis en suspension dans 480 ml de tampon Krebs Tris HCl 50 mM pH 7,7.

On fait ensuite incuber pendant 10 minutes à 37 °C, 2 ml de suspension en présence de 3H dihydroalprénolol à la concentration $10^{-9}$ M, i) seule, ii) avec des concentrations croissantes du produit à tester ou iii) pour déterminer la fixation non spécifique avec du propranolol non radioactif à la concentration $10^{-5}$ M.

Les suspension incubées sont filtrées sur Whatman GF/C et les filtres sont lavés par trois fois 5 ml de tampon Krebs Tris HCl pH 7,7 à 0 °C.

La radioactivité des filtres est mesurée par scintillation liquide.

L'affinité du produit testé pour les récepteurs béta$_1$ adrénergiquesest donnée relativement au propanolol comme produit de référence.

CD = concentration de propanolol inhibant 50 % de la fixation spécifique du 3H dihydroalprénolol.

CX = Concentration du produit à tester inhibant 50 % de la fixation spécifique du 3H dihydroalprénolol.

L'affinité relative est donnée par la relation ARL = 100 $\frac{CD}{CX}$

On a obtenu les résultats suivants :

| Produit de l'exemple | ARL en % |
|---|---|
| 1 | 28 |
| 7 | 44 |
| 21 | 20 |

On constate que certains produits de la demande possèdent une notable affinité pour les récepteurs béta$_1$ adrénergiques.

2) Affinité pour les récepteurs béta$_2$ adrénergiques

La technique est inspirée de celle de Möhler et Okada : Science, Vol. 198 p. 849-851 (1977).

On homogénéise dans 90 ml de sucrose 0,32 M, les cervelets prélevés sur des cerveaux de rats mâles pesant 150 g en moyenne. Après centrifugation à 1000 g du mélange homogénéisé pendant 20 minutes à 0 °C, le surnageant est centrifugé à 30 000 g pendant 15 minutes à 0, +4 °C.

Le culot est mis en suspension dans 120 ml de tampon Tris HCl 50mM pH 7,7 et centrifugé à 30 000 g pendant 15 minutes à 0, + 4 °C. Le nouveau culot obtenu est mis en suspension dans 480 ml de tampon Krebs Tris HCl 50 mM pH 7,7.

On fait ensuite incuber pendant 10 minutes à 37 °C, 2 ml de suspension en présence de 3H dihydroalprénolol à la concentration $10^{-9}$ M, i) seule, ii) avec des concentrations croissantes du produit à tester ou iii) pour déterminer la fixation non spécifique avec du propranolol non radioactif à la concentration $10^{-5}$ M.

Les suspension incubées sont filtrées sur Whatman GF/C et les filtres sont lavés par trois fois 5 ml de tampon Krebs Tris HCl pH 7,7 à 0 °C.

La radioactivité des filtres est mesurée par scintillation liquide.

L'affinité du produit testé pour les récepteurs béta$_2$ adrénergiquesest donnée relativement au propanolol comme produit de référence.

CD = concentration de propanolol inhibant 50 % de la fixation spécifique du 3H dihydroalprénolol.
CX = Concentration du produit à tester inhibant 50 % de la fixation spécifique du 3H dihydroalprénolol.

L'affinité relative est donnée par la relation ARL = 100 $\frac{CD}{CX}$

On a obtenu les résultats suivants :

| Produit de l'exemple | ARL en % |
|---|---|
| 1 | 15 |
| 7 | 81 |
| 21 | 27 |

On constate que certains produits de la demande possèdent une notable affinité pour les récepteurs béta$_2$ adrénergiques.

3) Action antiarythmique chez le rat

On trachéotomise des rats mâles pesant 300-350 g anesthésiés par voie intrapéritonéale à l'aide de 1,20 g/kg d'uréthane et les soumet à une respiration artificielle (40-50 insufflations de 3 ml/minute).

On implante des aiguilles en sous cutané de manière à enregistrer l'électrocardiogramme des rats sur le signal en dérivation DII.

On administre les produits à tester par voie intraveineuse.

Cinq minutes après l'administration du produit par voie intraveineuse ou 1 heure après administration par voie orale, on perfuse la veine jugulaire des rats avec 10 µg/mn sous 0,2 ml d'une solution d'aconitine et on note le temps d'apparition des troubles du rythme cardiaque.

Les résultats sont exprimés en pourcentage d'allongement du temps d'apparition des troubles du rythme cardiaque par rapport aux témoins et en fonction de la dose du produit testé.

Les résultats figurant sur le tableau ci-après montrent que les produits de la présente demande sont doués de bonnes propriétés antiarythmiques.

| Produit de l'exemple | Dose en mg/kg | Pourcentage d'allongement du temps |
|---|---|---|
| 1 | 1 mg/kg | 14 % |
| | 2,5 mg/kg | 54 % |
| | 5 mg/kg | 95 % |
| 3 | 2,5 mg/kg | 38 % |
| | 5 mg/kg | 99 % |
| | 10 mg/kg | 147 % |
| 4 | 1 mg/kg | 24 % |
| | 2,5 mg/kg | 34 % |
| | 5 mg/kg | 83 % |
| 5 | 0,5 mg/kg | 28 % |
| | 1 mg/kg | 43 % |
| | 2,5 mg/kg | 71 % |
| | 5 mg/kg | 97 % |

| Produit de l'exemple | Dose en mg/kg | Pourcentage d'allongement du temps |
|---|---|---|
| 6 | 5 mg/kg | 54 % |
| | 10 mg/kg | 99 % |
| 7 | 2,5 mg/kg | 12 % |
| | 5 mg/kg | 43 % |
| | 10 mg/kg | 125 % |
| 9 | 1 mg/kg | 25 % |
| | 2,5 mg/kg | 59 % |
| 10 | 1 mg/kg | 47 % |
| | 5 mg/kg | 116 % |
| 11 | 2,5 mg/kg | 42 % |
| | 5 mg/kg | 76 % |
| | 10 mg/kg | 173 % |
| 13 | 1 mg/kg | 21 % |
| | 2,5 mg/kg | 50 % |
| 14 | 1 mg/kg | 34 % |
| | 2,5 mg/kg | 57 % |
| | 5 mg/kg | 156 % |
| 15 | 1 mg/kg | 29 % |
| | 2,5 mg/kg | 86 % |
| 16 | 1 mg/kg | 49 % |
| | 2,5 mg/kg | 83 % |
| 17 | 1 mg/kg | 50 % |
| | 2,5 mg/kg | 90 % |
| | 5 mg/kg | 167 % |

| Produit de l'exemple | Dose en mg/kg | Pourcentage d'allongement du temps |
|---|---|---|
| 18 | 2,5 mg/kg | 62 % |
| | 5 mg/kg | 142 % |
| | 10 mg/kg | 193 % |
| 19 | 2,5 mg/kg | 67 % |
| | 5 mg/kg | 127 % |
| | 10 mg/kg | 203 % |
| 20 | 5 mg/kg | 44 % |
| | 10 mg/kg | 67 % |
| 21 | 2,5 mg/kg | 53 % |
| | 5 mg/kg | 79 % |

4/. Test d'activité anticalcique in vitro :

Des artères caudales de rat découpées en spirale sont reliées à des capteurs de tension et sont maintenues dans des cuves de 25 ml de tampon Krebs-bicarbonate de sodium (NaCl : 120,8 mM, KCl : 5,9 mM, $MgCl_2$ : 1,2 mM, $NaH_2PO_4$ : 1,2 mM, $NaHCO_3$ : 15,5 mM, glucose : 12,6 mM) à 37°C gazée avec un mélange $O_2$ : 95 % - $CO_2$ : 5%.

Les préparations sont dépolarisées par une solution tampon à concentration 100 mM en ions $K^+$ (NaCl : 26,7 mM, KCl : 100 mM, $MgCl_2$ : 1,2 mM, $NaH_2PO_4$ : 1,2 mM, $NaHCO_3$ : 15,5 mM, glucose : 12,6 mM).

On ajoute, sous un volume de 250 µl, du chlorure de calcium, de manière à obtenir une gamme de concentrations croissantes en ions $Ca^{2+}$ allant de 0,1 à 3,0 mM ; on enregistre les contractions de artères et établit ainsi une gamme témoin. On répète l'opération avec la gamme d'ions $Ca^{+2}$ toutes les 15 minutes et la préparation est lavée quatre fois, après chaque gamme.

Lorsque l'on obtient une réponse stable, l'on effectue l'opération avec les gammes d'ions $Ca^{2+}$ en présence de différentes concentrations du produit à tester, jusqu'à ce qu'une réponse stable soit obtenue.

Le contractions des artères dépendent de l'entrée des ions $Ca^{2+}$ dans les cellules des muscles lisses et sont provoquées par la dépolarisation du muscle lisse par les ions $K^+$ et par l'action de la noradrénaline libérée au niveau présynaptique. En recommençant l'opération avec des artères dénervées par action de 6-OH dopamine, on supprime l'action propre due à la noradrénaline.

Les résultats sont exprimés en CI 50 (concentration inhibitrice 50) concentration du produit testé qui inhibe de 50 % la contraction due aux ions $K^+$.

On constate d'après les résultats figurant sur tableau ci-après que les produits de la présente demande possèdent une forte activité anticalcique.

| Produit de l'exemple | CI 50 en µM |
|---|---|
| 1 | 2,5 |
| 3 | 8 |
| 4 | 1,8 |
| 5 | 3,9 |
| 6 | 6,3 |
| 7 | 8,8 |
| 8 | 9 |
| 9 | 0,44 |
| 13 | 8,4 |
| 19 | 0,25 |
| 20 | 2,8 |
| 22 | 3 |

5) Etude de la toxicité aigüe

On a évalué les doses létales $DL_0$ des différents composés testés après administration par voie orale chez la souris.

On appelle $DL_0$ la dose maximale ne provoquant aucune mortalité 5 en 8 jours.

Les résultats obtenus sont les suivants :

| Produit de l'exemple | $DL_0$ en mg/kg |
|---|---|
| 1 | > 400 |
| 3 | 80 |
| 5 | 100 |
| 6 | > 400 |
| 7 | 80 |
| 17 | 200 |
| 19 | 400 |

6/. Etude de l'activité hypotensive sur le rat normotendu anesthésié du produit de l'exemple 19.

Des rats mâles Sprague Dawley (CR) sont anesthésiés par voir intrapéritonéale au pentobarbital sodique (60 mg/kg).

Une veine jugulaire est cathétérisée pour l'injection du produit, et une artère carotide est cathétérisée pour l'enregistrement de la pression artérielle.

Le produit à tester est mis en solution dans 10% d'éthanol puis injecté sous un volume de 1 cm3/kg.

La pression est notée aux temps 5 minutes et 30 minutes après l'injection du produit.

Le tableau ci-après indique les variations exprimées en pourcentage de la pression artérielle après administration du produit testé par rapport à la pression artérielle témoin initiale.

Résultats :

| dose | 5mn après administration | 30 mn après administration |
|---|---|---|
| 10 mg/kg | - 55 | - 13 |

**Revendications**

**Revendications pour les Etats contractants suivants : AT, BE, CH, DE, FR, GB, IT, LI, LU, NL, SE**

**1.** Nouveaux composés de formule (I) :

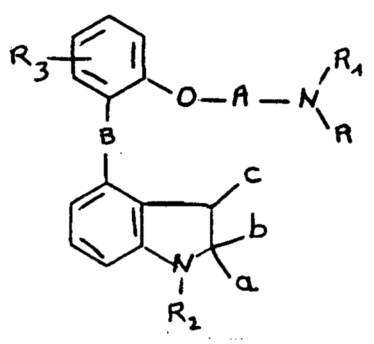

(I)

dans laquelle

- soit $R_2$ et $R_3$ représentent chacun un atome d'hydrogène, B représente une chaîne -CONH-, NH étant du côté de l'indole, b représente un atome d'hydrogène, a et c forment ensemble une seconde liaison entre les carbones qui les portent, A représente une chaîne

$$-CH_2-\underset{\displaystyle OH}{\underset{|}{C}H}-CH_2-$$

et ou bien $R_1$ représente un atome d' hydrogène et R représente un radical choisi parmi les radicaux 1,1-diméthylpropyle,

$$-\underset{\displaystyle CH_3}{\overset{\displaystyle CH_3}{\overset{|}{\underset{|}{C}}}}-C\equiv C-,\quad -\underset{\displaystyle CH_3}{\overset{\displaystyle CH_3}{\overset{|}{\underset{|}{C}}}}-CH_2-\underset{\displaystyle CH_3}{\overset{\displaystyle CH_3}{\overset{|}{\underset{|}{C}}}}-CH_3 \text{ ou } -\underset{\displaystyle CH_3}{\overset{\displaystyle CH_3}{\overset{|}{\underset{|}{C}}}}-CH_2OH$$

ou bien R et $R_1$ forment ensemble, avec l'atome d'azote auquel ils sont liés, un noyau

morpholine,

- soit $R_2$ et $R_3$ représentent chacun un atome d'hydrogène, B représente une chaîne -CO-NH, NH étant du côté de l'indole, b représente un atome d'hydrogène et a et c forment ensemble une seconde liaison entre les carbones qui les portent, A représente une chaîne $-(CH_2)_3-$, $R_1$ représente un atome d'hydrogène et R un radical choisi parmi les radicaux 1,1-diméthylpropyle, cyclohexyle, cyclohexylméthyle, propyle, isopropyle ou

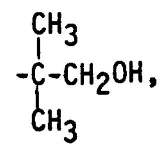

- soit $R_2$ et $R_3$ représentent chacun un atome d'hydrogène, B représente -NH-CO,

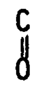

étant du côté de l'indole, b représente un atome d'hydrogène et a et c forment ensemble une seconde liaison entre les carbone qui les portent, A représente la chaîne $-(CH_2)_3-$, $R_1$ représente un atome d'hydrogène et R représente un radical choisi parmi les radicaux cyclopentyle, cyclohexyle, 1,1-diméthylpropyle ou

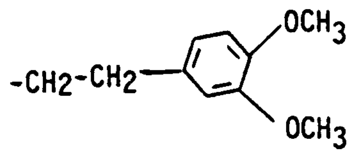

- soit $R_2$ et $R_3$ représentent un atome d'hydrogène, B représente -NH-CO-, -NH étant du côté de l'indole, b représente un atome d'hydrogène et a et c forment ensemble une seconde liaison entre les carbones qui les portent, A représente la chaîne $-(CH_2)_4-$, $R_1$ représente un atome d'hydrogène et R représente le groupement 1,1-diméthyléthyle,
- soit $R_2$ représente un radical méthyle et $R_3$ représente un atome d'hydrogène, B représente -NH-CO-, NH étant du côté de l'indole, b représente un atome d' hydrogène et a et c forment ensemble une seconde liaison entre les carbones qui les portent, A représente la chaîne

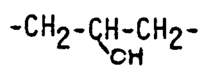

et R représente le groupement 1,1-diméthyléthyle,

- soit les substituants $R_1$ et R forment avec l'atome d'azote auquel ils sont attachés un groupement

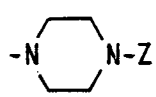

dans lequel Z représente un groupement

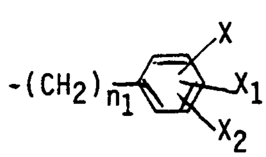

ou un groupement

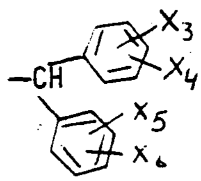

dans lesquels $n_1$ peut prendre les valeurs 1, 2 ou 3 et X, $X_1$, $X_2$, $X_3$, $X_4$, $X_5$ et $X_5$ identiques ou différents représentent un atome d'hydrogène, un radical alkyle renfermant de 1 à 5 atomes de carbone, un radical alkoxy renfermant de 1 à 3 atomes de carbone, un atome d'halogène, un radical nitro, amino, monoalkylamino ou dialkylamino, à la condition que X, $X_1$ et $X_2$ ne représentent pas tous trois un atome d'hydrogène, A représente une chaîne $-(CH_2)_n-$ dans laquelle n peut prendre les valeurs 2, 3, 4 ou 5 ou une chaîne

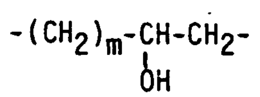

dans laquelle m peut prendre les valeurs 1, 2 ou 3, B représente une chaîne -CO-NH- ou -NH-CO-, $R_3$ représente un atome d'hydrogène, un radical alkyle renfermant de 1 à 5 atomes de carbone, un radical alkoxy renfermant de 1 à 3 atomes de carbone, un atome de chlore, de brome ou d'iode, un radical nitro ou un radical amino éventuellement substitué par un groupe acyle aliphatique renfermant de 2 à 5 atomes de carbone ou par un radical alkyle renfermant de 1 à 5 atomes de carbone, a représente ensemble avec b une fonction oxo, ou représente ensemble avec c une seconde liaison entre les carbone qui les portent, b représente un atome d'hydrogène, ou ensemble avec a une fonction oxo, c représente un atome d'hydrogène, ou ensemble avec a représente une seconde liaison entre les carbones qui les portent, $R_2$ représente un atome d'hydrogène, un radical alkyle linéaire renfermant de 1 à 5 atomes de carbone ou un radical alkyle ramifié renfermant de 3 à 5 atomes de carbone ou un radical alkényle ou alkynyle renfermant de 2 à 5 atomes de carbone ou un radical aralkyle renfermant de 7 à 12 atomes de carbone éventuellement substitué par 1, 2 ou 3 radicaux choisis dans le groupe constitué par les atomes d'halogène et les radicaux méthyle, éthyle, méthoxy, éthoxy, trifluorométhyle, méthylthio, amino et nitro ou un radical cycloalkylalkyle renfermant de 4 à 7 atomes de carbone, ainsi que leurs sels d'addition avec les acides minéraux ou organiques.

**2.** Composés de formule (I), tels que définis à la revendication 1, dans laquelle les substituants $R_1$ et R forment avec l'atome d'azote auquel ils sont liés un groupement

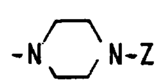

dans lequel Z a la signification précédente, A représente une chaîne

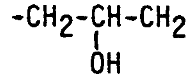

ou $-(CH_2)_3-$ ou $-(CH_2)_4-$, $R_3$ représente un atome d'hydrogène, $R_2$ représente un atome d'hydrogène ou un radical méthyle, b représente un atome d'hydrogène et a et c forment ensemble une seconde liaison entre les carbones qui les portent ainsi que leurs sels d'addition avec les acides minéraux ou organiques.

**3.** Le 2-[3-(1,1-diméthylpropyl) amino] 2-hyroxypropoxy] N-(1H-indol-4-yl) benzamide et son benzoate et son oxalate neutre,

**4.** Le 2-[3-[(1,1-diméthyléthyl) amino] 2-hydroxypropoxy] N-(1-méthyl 1H-indol-4-yl) benzamide et son chlorhydrate,

**5.** Le 2-[2-hydroxy 3-[(1,1,3,3-tétraméthylbutyl)amino] propoxy] N-(1H-indol-4-yl) benzamide, et
- le 2-[2-hydroxy 3-[4-(diphénylméthyl) 1-pipérazinyl] propoxy] N-(1H-indol-4-yl) benzamide et son oxalate neutre.

**6.** Procédé de préparation des dérivés tels que définis par la formule (I) de la revendication 1, ainsi que de leurs sels, caractérisé en ce que l'on fait réagir un dérivé de formule (II) :

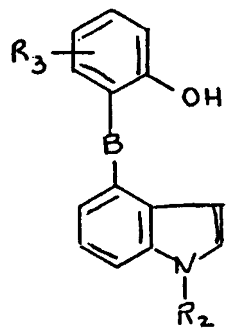

(II)

dans laquelle B et $R_3$ ont la signification déjà indiquée et $R_2$ représente un atome d'hydrogène ou un méthyle,
soit avec un halogénure de formule (III) :

Hal-G (III)

dans laquelle Hal représente un atome de chlore, de brome ou d'iode et -G représente un groupement de formule $-(CH_2)_n$-D, dans laquelle D représente un atome de chlore, de brome, d'iode, ou un radical hydroxy ou un sulfonate de ce radical hydroxy, et n a la signification déjà indiquée, ou -G représente un groupement

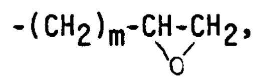

dans laquelle m a la signification déjà indiquée, pour obtenir un dérivé de formule (IV) :

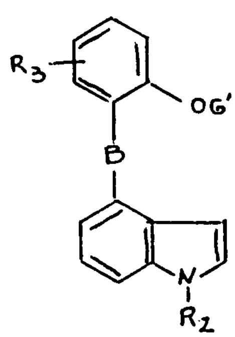

(IV)

dans laquelle B, $R_2$ et $R_3$ ont la signification déjà indiquée, et G' représente un groupement de formule $-(CH_2)_n$-Hal dans laquelle n et Hal ont la signification déjà indiquée, ou un groupement de formule

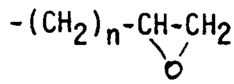

défini ci-dessus, que l'on fait réagir avec une amine de formule (V) :

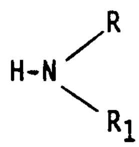

(V)

dans laquelle R et $R_1$ ont la signification déjà indiquée, pour obtenir un produit de formule ($I_A$) :

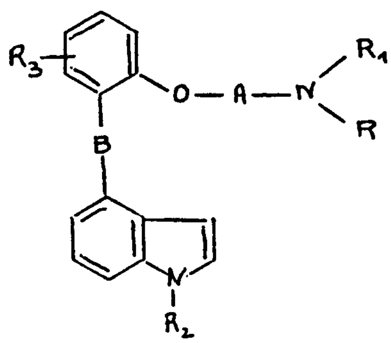

($I_A$)

dans laquelle A, B, R, $R_1$, $R_2$ et $R_3$ ont la signification déjà indiquée, que ou bien l'on isole et, si désiré, salifie, ou bien dans le cas où dans le produit de formule ($I_A$), R et $R_1$ forment avec l'atome d'azote auquel ils sont liés un groupement

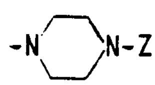

dans lequel Z a la signification déjà indiquée, l'on soumet le produit de formule ($I_B$) correspondant :

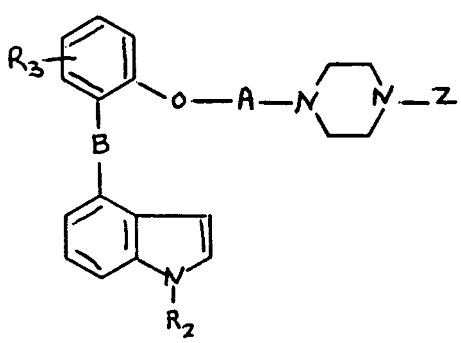

($I_B$)

dans laquelle A, B, $R_2$, $R_3$ et Z ont les significations déjà indiquées, à l'action d'un agent d'halogénation pour obtenir un produit de formule (VII) :

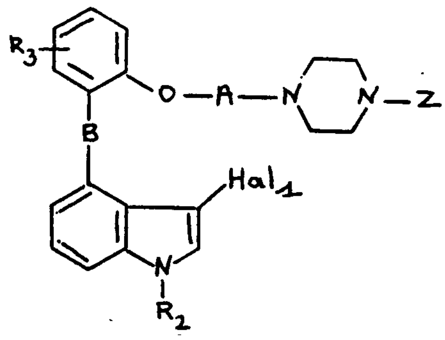

(VII)

dans laquelle A, B, $R_2$, $R_3$ et Z ont les significations déjà indiquées et $Hal_1$ représente un atome de brome ou de chlore, que l'on soumet à une hydrolyse pour obtenir un composé de formule ($I_C$) :

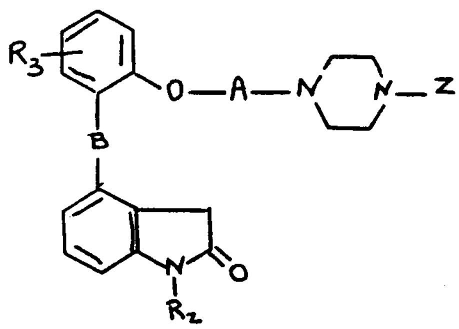

($I_C$)

dans laquelle A, B, $R_2$, $R_3$ et Z ont les significatins déjà indiquées, que l'on isole et si désiré salifie, soit l'on fait réagir ledit produit de formule (II) avec un dérivé de formule (VI) :

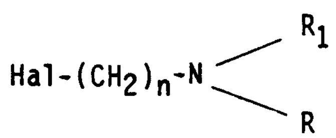

(VI)

dans laquelle Hal, n, R et $R_1$ ont la signification déjà indiquée, pour obtenir le dérivé de formule ($I_A$) dans laquelle A représente une chaîne $-(CH_2)n$, que l'on isole et si désiré, salifie, ou dans le cas où dans le produit de formule ($I_A$), R et $R_1$ forment avec l'atome d'azote auquel ils sont attachés, un groupement

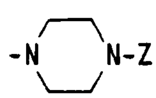

dans lequel Z a la signification déjà indiquée l'on soumet le produit de formule ($I_B$) correspondant à un agent d'halogénation puis à un réactif d'hydrolyse pour obtenir le produit de formule ($I_C$) que l'on isole et si désiré salifie.

7. Médicaments, caractérisés en ce qu'ils sont constitués par les nouveaux dérivés du benzamide tels que définis par la revendication 1, ainsi que leurs sels d'addition avec les acides pharmaceutiquement acceptables.

8. Médicaments, caractérisés en ce qu'ils sont constitués par les nouveaux dérivés du benzamide tels que définis à l'une quelconque des revendications 2 à 5, ainsi que leurs sels d'addition avec les acides pharmaceutiquement acceptables.

9. Compositions pharmaceutiques, caractérisées en ce qu'elles renferment, à titre de principe actif, l'un au moins des médicaments tels que définis à l'une des revendications 7 ou 8.

10. A titre de composés industriels nouveaux, les composés de formule (VII) tels que définis à la revendication 6.

**Revendications pour l'Etat contractant suivant: ES**

1. Procédé de préparation des nouveaux composés de formule (I) :

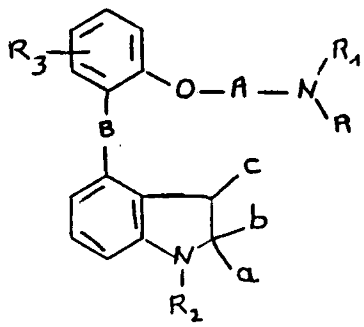

(I)

dans laquelle

- soit $R_2$ et $R_3$ représentent chacun un atome d'hydrogène, B représente une chaîne -CONH-, NH étant du côté de l'indole, b représente un atome d'hydrogène, a et c forment ensemble une seconde liaison entre les carbones qui les portent, A représente une chaîne

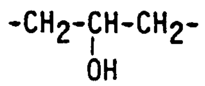

et ou bien $R_1$ représente un atome d'hydrogène et R représente un radical choisi parmi les radicaux 1,1-diméthylpropyle,

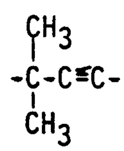 , 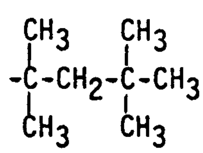 ou 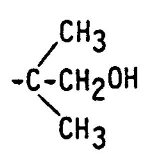

ou bien R et $R_1$ forment ensemble, avec l'atome d'azote auquel ils sont liés, un noyau morpholine,

- soit $R_2$ et $R_3$ représentent chacun un atome d'hydrogène, B représente une chaîne -CO-NH, NH étant du côté de l'indole, b représente un atome d'hydrogène et a et c forment ensemble une seconde liaison entre les carbones qui les portent, A représente une chaîne $-(CH_2)_3-$, $R_1$ représente un atome d'hydrogène et R un radical choisi parmi les radicaux 1,1-diméthylpropyle, cyclohexyle, cyclohexylméthyle, propyle, isopropyle ou

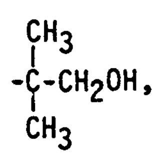

- soit $R_2$ et $R_3$ représentent chacun un tome d'hydrogène, B représente -NH-CO,

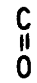

étant du côté de l'indole, b représente un atome d'hydrogène et a et c forment ensemble une seconde liaison entre les carbone qui les porten, A représente la chaîne $-(CH_2)_3-$, $R_1$ représente un atome d'hydrogène et R représente un radical choisi parmi les radicaux cyclopentyle, cyclohexyle, 1,1-diméthylpropyle ou

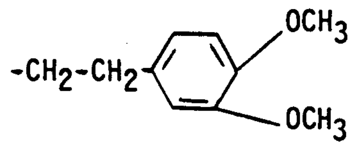

- soit $R_2$ et $R_3$ représentent un atome d'hydrogène, B représente -NH-CO-, -NH étant du côté de l'indole, b représente un atome d'hydrogène et a et c forment ensemble une seconde liaison entre les carbones qui les portent, A représente la chaîne $-(CH_2)_4-$, $R_1$ représente un atome d'hydrogène et R représente le groupement 1,1-diméthyléthyle,
- soit $R_2$ représente un radical méthyle et $R_3$ représente un atome d'hydrogène, B représente -NH-CO-, NH étant du côté de l'indole, b représente un atome d'hydrogène et a et c forment ensemble une seconde liaison entre les carbones qui les portent, A représente la chaîne

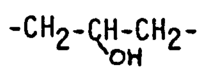

et R représente le groupement 1,1-diméthyléthyle,
- soit les substituants $R_1$ et R forment avec l'atome d'azote auquel ils sont attachés un groupement

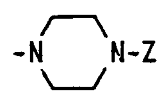

dans lequel Z représente un groupement

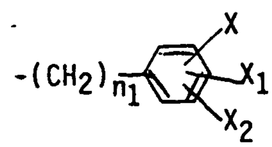

ou un groupement

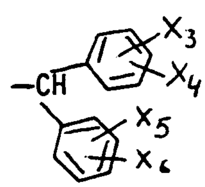

dans lesquels $n_1$ peut prendre les valeurs 1, 2 ou 3 et X, $X_1$, $X_2$, $X_3$, $X_4$, $X_5$ et $X_5$ identiques ou différents représentent un atome d'hydrogène, un radical alkyle renfermant de 1 à 5 atomes de carbone, un radical alkoxy renfermant de 1 à 3 atomes de carbone, un atome d'halogène, un radical nitro, amino, manoalkylamino ou dialkylamino, à la condition que X, $X_1$ et $X_2$ ne

représentent pas tous trois un atome d'hydrogène, A représente une chaîne $-(CH_2)_n-$ dans laquelle n peut prendre les valeurs 2, 3, 4 ou 5 ou une chaîne

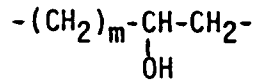

dans laquelle m peut prendre les valeurs 1, 2 ou 3, B représente une chaîne -CO-NH- ou -NH-CO-, $R_3$ représente un atome d'hydrogène, un radical alkyle renfermant de 1 à 5 atomes de carbone, un radical alkoxy renfermant de 1 à 3 atomes de carbone, un atome de chlore, de brome ou d'iode, un radical nitro ou un radical amino éventuellement substitué par un groupe acyle aliphatique renfermant de 2 à 5 atomes de carbone ou par un radical alkyle renfermant de 1 à 5 atomes de carbone, a représente ensemble avec b une fonction oxo, ou représente ensemble avec c une seconde liaison entre les carbone qui les portent, b représente un atome d'hydrogène, ou ensemble avec a une fonction oxo, c représente un atome d'hydrogène, ou ensemble avec a représente une seconde liaison entre les carbones qui les portent, $R_2$ représente un atome d'hydrogène, un radical alkyle linéaire renfermant de 1 à 5 atomes de carbone ou un radical alkyle ramifié renfermant de 3 à 5 atomes de carbone ou un radical alkényle ou alkynyle renfermant de 2 à 5 atomes de carbone ou un radical aralkyle renfermant de 7 à 12 atomes de carbone éventuellement substitué par 1, 2 ou 3 radicaux choisis dans le groupe constitué par les atomes d'halogène et les radicaux méthyle, éthyle, méthoxy, éthoxy, trifluorométhyle, méthylthio, amino et nitro ou un radical cycloalkylalkyle renfermant de 4 à 7 atomes de carbone, ainsi que leurs sels d'addition avec les acides minéraux ou organiques, caractérisé en ce que l'on fait réagir un dérivé de formule (II) :

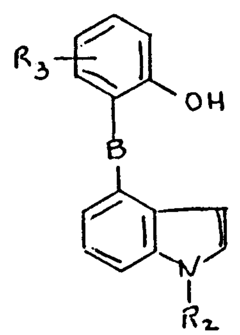

(II)

dans laquelle B et $R_3$ ont la signification déjà indiquée et $R_2$ représente un atome d'hydrogène ou un méthyle,
soit avec un halogénure de formule (III) :

Hal-G (III)

dans laquelle Hal représente un atome de chlore, de brome ou d'iode et -G représente un groupement de formule $-(CH_2)_n-D$, dans laquelle D représente un atome de chlore, de brome, d'iode, ou un radical hydroxy ou un sulfonate de ce radical hydroxy, et n a la signification déjà indiquée, ou -G représente un groupement

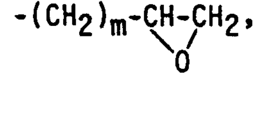

dans laquelle m a la signification déjà indiquée, pour obtenir un dérivé de formule (IV) :

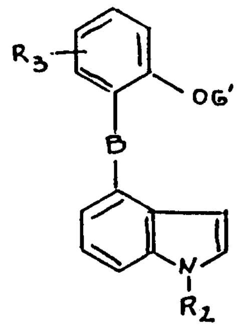

(IV)

dans laquelle B, $R_2$ et $R_3$ ont la signification déjà indiquée, et G' représente un groupement de formule $-(CH_2)_n$-Hal dans laquelle n et Hal ont la signification déjà indiquée, ou un groupement de formule

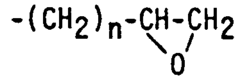

défini ci-dessus, que l'on fait réagir avec une amine de formule (V) :

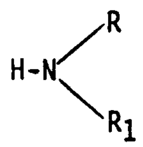

(V)

dans laquelle R et $R_1$ ont la signification déjà indiquée, pour obtenir un produit de formule ($I_A$) :

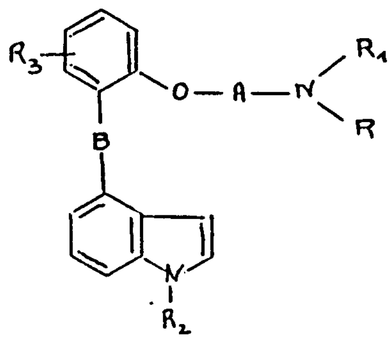

($I_A$)

dans laquelle A, B, R, $R_1$, $R_2$ et $R_3$ ont la signification déjà indiquée, que ou bien l'on isole et, si désiré, salifie, ou bien dans le cas où dans le produit de formule ($I_A$), R et $R_1$ forment avec l'atome d'azote auquel ils sont liés un groupement

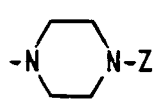

dans lequel Z a la signification déjà indiquée, l'on soumet le produit de formule ($I_B$) correspondant :

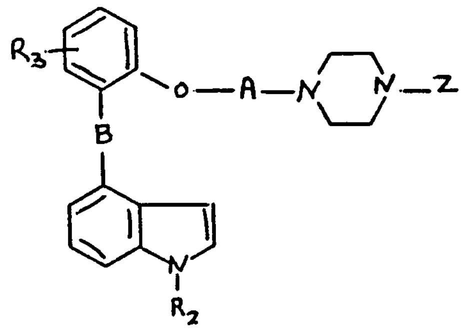

($I_B$)

dans laquelle A, B, $R_2$, $R_3$ et Z ont les significations déjà indiquées, à l'action d'un agent d'halogénation pour obtenir un produit de formule (VII) :

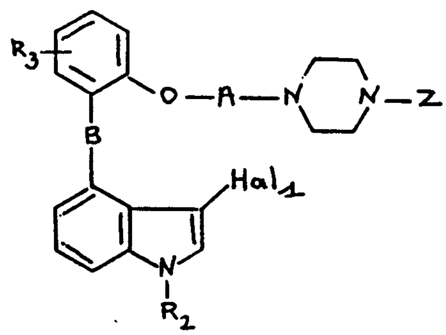

(VII)

dans laquelle A, B, $R_2$, $R_3$ et Z ont les significations déjà indiquées et $Hal_1$ représente un atome de brome ou de chlore, que l'on soumet à une hydrolyse pour obtenir un composé de formule ($I_C$) :

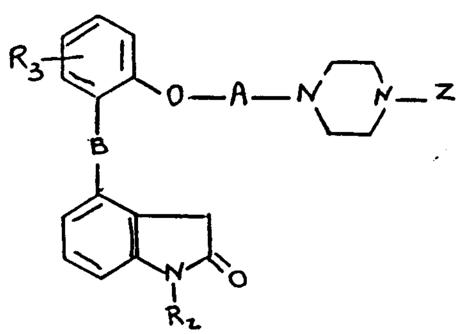

($I_C$)

dans laquelle A, B, $R_2$, $R_3$ et Z ont les significations déjà indiquées, que l'on isole et si désiré salifie,

soit l'on fait réagir ledit produit de formule (II) avec un dérivé de formule (VI) :

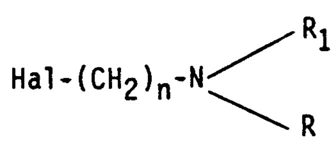

(VI)

dans laquelle Hal, n, R et $R_1$ ont la signification déjà indiquée, pour obtenir le dérivé de formule ($I_A$) dans laquelle A représente une chaîne $-(CH_2)_n$, que l'on isole et si désiré, salifie, ou dans le cas où dans le produit de formule ($I_A$), R et $R_1$ forment avec l'atome d'azote auquel ils sont attachés, un groupement

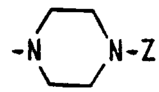

dans lequel Z a la signification déjà indiquée l'on soumet le produit de formule ($I_B$) correspondant à un agent d'halogénation puis à un réactif d'hydrolyse pour obtenir le produit de formule ($I_C$) que l'on isole et si désiré salifie.

**2.** Procédé selon la revendication 1, caractérisé en ce que l'on utilise au départ un produit de formule (II) dans laquelle $R_3$ représente un atome d'hydrogène, $R_2$ représente un atome d'hydrogène ou un radical méthyle que l'on soumet à un halogénure de formule (III) Hal-G dans laquelle G représente un groupement $-(CH_2)_nD$ dans lequel n est 2 ou 3 ou un groupement

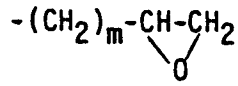

dans lequel m est 1, et un produit de formule (V) ou (VI) dans laquelle $R_1$ et R forment avec l'atome d'azote auquel ils sont liés un groupement

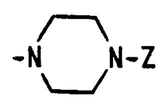

dans lequel Z a la signification indiquée à la revendication 1.

**Revendications pour l'Etat contractant suivant: GR**

**1.** Procédé de préparation des noveaux composés de formule (I) :

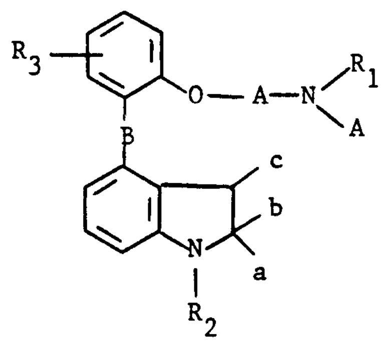

(I)

dans laquelle

- soit $R_2$ et $R_3$ représentent chacun un atome d'hydrogène, B représente une chaîne -CONH-, NH étant du côté de l'indole, b représente un atome d'hydrogène, a et c forment ensemble une seconde liaison ente les carbones qui les portent, A représente une chaîne

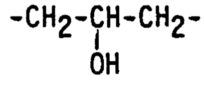

et ou bien $R_1$ représente un atome d'hydrogène et R représente un radical choisi parmi les radicaux 1,1-diméthylpropyle,

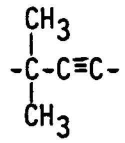, 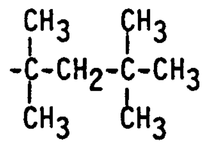 ou 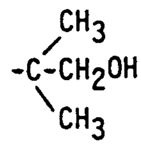

ou bien R et $R_1$ forment ensemble, avec l'atome d'azote auquel ils sont liés, un noyau morpholine,

- soit $R_2$ et $R_3$ représentent chacun un atome d'hydrogène, B représente une chaîne -CO-NH, NH étant du côté de l'indole, b représente un atome d'hydrogène et a et c forment ensemble une seconde liaison entre les carbones qui les portent, A représente une chaîne $-(CH_2)_3-$, $R_1$ représente un atome d'hydrogène et R un radical choisi parmi les radicaux 1,1-diméthylpropyle, cyclohexyle, cyclohexylméthyle, propyle, isopropyle ou

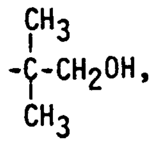

- soit $R_2$ et $R_3$ représentent chacun un atome d'hydrogène, B représente -NH-CO,

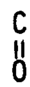

étant du côté de l'indole, b représente un atome d'hydrogène et a et c forment ensemble une seconde liaison entre les carbone qui les portent, A représente la chaîne $-(CH_2)_3-$, $R_1$ représente un atome d'hydrogène et R représente un radical choisi parmi les radicaux cyclopentyle, cyclohexyle, 1,1-diméthylpropyle ou

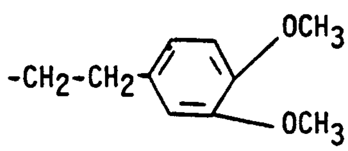

- soit $R_2$ et $R_3$ représentent un atome d' hydrogène, B représente -NH-CO-, -NH étant du côté de l'indole, b représente un atome d'hydrogène et a et c forment ensemble une seconde liaison entre les carbones qui les portent, A représente la chaîne $-(CH_2)_4-$, $R_1$ représente un atome d'hydrogène et R représente le groupement 1,1-diméthyléthyle,
- soit $R_2$ représente un radical méthyle et $R_3$ représente un atome d'hydrogène, B représente -NH-CO-, NH étant du côté de l'indole, b représente un atome d' hydrogène et a et c forment ensemble une seconde liaison entre les carbones qui les portent, A représente la chaîne

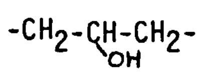

et R représente le groupement 1,1-diméthyléthyle, - soit les sùbstituants $R_1$ et R forment avec l'atome d'azote auquel ils sont attachés un groupement

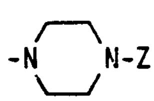

dans lequel Z représente un groupement

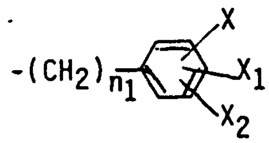

ou un groupement

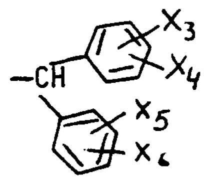

dans lesquels $n_1$ peut prendre les valeurs 1, 2 ou 3 et X, $X_1$, $X_2$, $X_3$, $X_4$, $X_5$ et $X_5$ identiques ou différents représentent un atome d'hydrogène, un radical alkyle renfermant de 1 à 5 atomes de carbone, un radical alkoxy renfermant de 1 à 3 atomes de carbone, un atome d'halogène, un radical nitro, amino, monoalkylamino ou dlalkylamino, à la condition que X, $X_1$ et $X_2$ ne représentent pas tous trois un atome d'hydrogène, A représente une chaîne $-(CH_2)_n-$ dans laquelle n peut prendre les valeurs 2, 3, 4 ou 5 ou une chaîne

$$-(CH_2)_m-\underset{OH}{\underset{|}{CH}}-CH_2-$$

dans laquelle m peut prendre les valeurs 1, 2 ou 3, B représente une chaîne -CO-NH- ou -NH-CO-, $R_3$ représente un atome d'hydrogène, un radical alkyle renfermant de 1 à 5 atomes de carbone, un radical alkoxy renfermant de 1 à 3 atomes de carbone, un atome de chlore, de brome ou d'iode, un radical nitro ou un radical amino éventuellement substitué par un groupe acyle aliphatique renfermant de 2 à 5 atomes de carbone ou par un radical alkyle renfermant de 1 à 5 atomes de carbone, a représente ensemble avec b une fonction oxo, ou représente ensemble avec c une seconde liaison entre les carbone qui les portent, b représente un atome d'hydrogène, ou ensemble avec a une fonction oxo, c représente un atome d'hydrogène, ou ensemble avec a représente une seconde liaison entre les carbones qui les portent, $R_2$ représente un atome d'hydrogène, un radical alkyle linéaire renfermant de 1 à 5 atomes de carbone ou un radical alkyle ramifié renfermant de 3 à 5 atomes de carbone ou un radical alkényle ou alkynyle renfermant de 2 à 5 atomes de carbone ou un radical aralkyle renfermant de 7 à 12 atomes de carbone éventuellement substitué par 1, 2 ou 3 radicaux choisis dans le groupe constitué par les atomes d'halogène et les radicaux méthyle, éthyle, méthoxy, éthoxy, trifluorométhyle, méthylthio, amino et nitro ou un radical cycloalkylalkyle renfermant de 4 à 7 atomes de carbone, ainsi que leurs sels d'addition avec les acides minéraux ou organiques, caractérisé en ce que l'on fait réagir un dérivé de formule (II) :

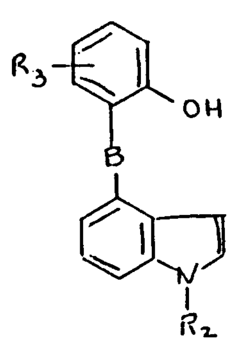

(II)

dans laquelle B et $R_3$ ont la signification déjà indiquée et $R_2$ représente un atome d'hydrogène

ou un méthyle,
soit avec un halogénure de formule (III) :

Hal-G (III)

dans laquelle Hal représente un atome de chlore, de brome ou d'iode et -G représente un groupement de formule $-(CH_2)_n$-D, dans laquelle D représente un atome de chlore, de brome, d'iode, ou un radical hydroxy ou un sulfonate de ce radical hydroxy, et n a la signification déjà indiquée, ou -G représente un groupement

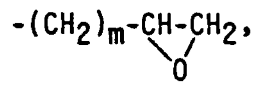

dans laquelle m a la signification déjà indiquée, pour obtenir un dérivé de formule (IV) :

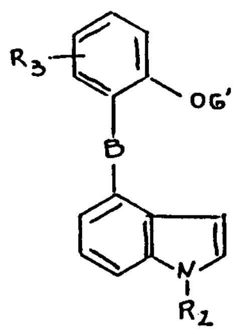

(IV)

dans laquelle B, $R_2$ et $R_3$ ont la signification déjà indiquée, et G' représente un groupement de formule $-(CH_2)_n$-Hal dans laquelle n et Hal ont la signification déjà indiquée, ou un groupement de formule

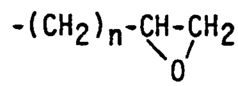

défini ci-dessus, que l'on fait réagir avec une amine de formule (V) :

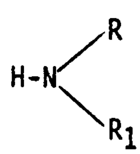

(V)

dans laquelle R et $R_1$ ont la signification déjà indiquée, pour obtenir un produit de formule ($I_A$) :

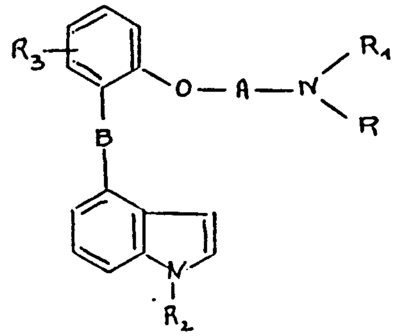

($I_A$)

dans laquelle A, B, R, $R_1$, $R_2$ et $R_3$ ont la signification déjà indiquée, que ou bien l'on isole et, si désiré, salifie, ou bien dans le cas où dans le produit de formule ($I_A$), R et $R_1$ forment avec l'atome d'azote auquel ils sont liés un groupement

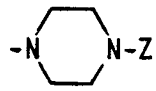

dans lequel Z a la signification déjà indiquée, l'on soumet le produit de formule ($I_B$) correspondant :

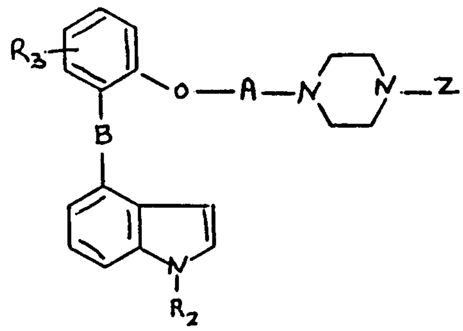

($I_B$)

dans laquelle A, B, $R_2$, $R_3$ et Z ont les significations déjà indiquées, à l'action d'un agent d'halogénation pour obtenir un produit de formule (VII) :

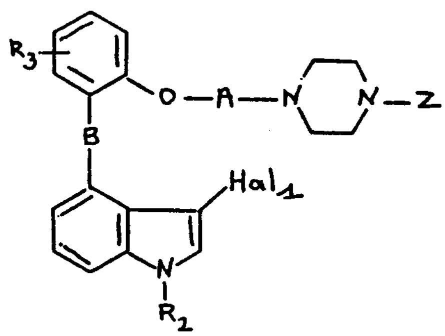

(VII)

dans laquelle A, B, $R_2$, $R_3$ et Z ont les significations déjà indiquées et $Hal_1$ représente un atome de brome ou de chlore, que l'on soumet à une hydrolyse pour obtenir un composé de formule ($I_C$) :

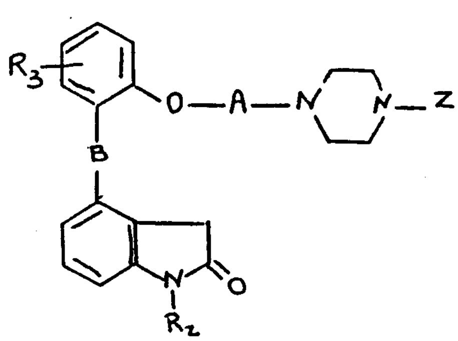

($I_C$)

dans laquelle A, B, $R_2$, $R_3$ et Z ont les significations déjà indiquées, que l'on isole et si désiré salifie,

soit l'on fait réagir ledit produit de formule (II) avec un dérivé de formule (VI) :

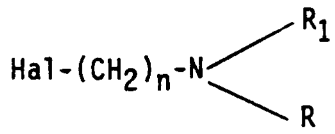 (VI)

dans laquelle Hal, n, R et $R_1$ ont la signification déjà indiquée, pour obtenir le dérivé de formule ($I_A$) dans laquelle A représente une chaîne $-(CH_2)_n$, que l'on isole et si désiré, salifie, ou dans le cas où dans le produit de formule ($I_A$), R et $R_1$ forment avec l'atome d'azote auquel ils sont attachés, un groupement

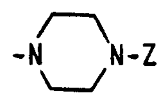

dans lequel Z a la signification déjà indiquée l'on soumet le produit de formule ($I_B$) correspondant à un agent d'halogénation puis à un réactif d'hydrolyse pour obtenir le produit de formule ($I_C$) que l'on isole et si désiré salifie.

**2.** Procédé selon la revendication 1, caractérisé en ce que l'on utilise au départ un produit de formule (II) dans laquelle $R_3$ représente un atome d'hydrogène, $R_2$ représente un atome d'hydrogène ou un radical méthyle que l'on soumet à un halogénure de formule (III) Hal-G dans laquelle G représente un groupement $-(CH_2)_nD$ dans lequel n est 2 ou 3 ou un groupement

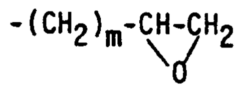

dans lequel m est 1, et un produit de formule (V) ou (VI) dans laquelle $R_1$ et R forment avec l'atome d'azote auquel ils sont liés un groupement

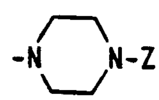

dans lequel Z a la signification indiquée à la revendication 1.

**3.** Procédé selon la revendication 1 ou 2, caractérisé en ce que l'on prépare un produit de formule (I) dans laquelle les substituants $R_1$ et R forment avec l'atome d'azote auquel ils liés un groupement

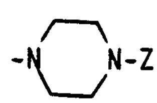

dans lequel Z a la signification précédente, A représente une chaîne

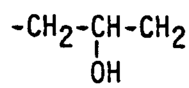

ou $-(CH_2)_3-$ ou $-(CH_2)_4-$, $R_3$ représente un atome d'hydrogène, $R_2$ représente un atome d'hydrogène ou un radical méthyle, b représente un atome d'hydrogène et a et c forment ensemble une seconde liaison entre les carbones qui les portent ainsi que leurs sels d'addition avec les acides minéraux ou organiques.

**4.** Procédé selon la revendication 1, caractérisé en ce que l'on prépare le 2-[3-[(1,1-diméthylpropyl) amino] 2-hydroxypropoxy] N-(1H-indol-4-yl) benzamide et son benzoate et son oxalate neutre.

**5.** Procédé selon la revendication 1, caractérisé en ce que l'on prépare le 2-[3-[(1,1-diméthyléthyl) amino] 2-hydroxypropoxy] N-(1-méthyl 1H-indol-4-yl) benzamide et son chlorhydrate.

**6.** Procédé selon la revendication 1, caractérisé en ce que l'on prépare le 2-[2-hydroxy 3-[(1,1,3,3-tétraméthylbutylamino] propoxy] N-(1H-indol-4-yl) benzamide.

**7.** Procédé selon la revendication 1, caractérisé en ce que l'on prépare le 2-[2-hydroxy 3-[4-(diphénylméthyl) 1-pipérazinyl] propoxy] N-(1H-indol-4-yl) benzamide et son oxalate neutre.

**8.** A titre de composés industriels nouveaux, les composés de formule (VII) tels que définis à la revendication 1.

**9.** Procédé de préparation de compositions pharmaceutiques, caractérisées en ce que l'on met à titre de principe actif l'un au moins des produits de formule (I), telle que définie à la revendication 1 ou l'un au moins de leurs sels d'addition avec les acides pharmaceutiquement acceptables, sous une forme destinée à cet emploi.

**10.** Procédé de préparation de compositions pharmaceutiques, caractérisées en ce que l'on met à titre de principe actif l'un au moins des produits de formule (I), telle que définie à la revendication 2 ou 3, ou l'un au moins de leurs sels d'addition avec les acides pharmaceutiquement acceptables, sous une forme destinée à cet emploi.

**11.** Procédé selon la revendication 4, caractérisé en ce que l'on utilise à titre de principe actif le 2-[3-[(1,1-diméthyléthyl) amino] 2-hydroxy propoxy] N-(1H-indol-4-yl) benzamide et son benzoate et son oxalate neutre.

**12.** Procédé selon la revendication 2 ou 3, caractérisé en ce que l'on utilise à titre de principe actif le 2-[3-[(1,1-diméthyléthyl) amino] 2-hydroxypropoxy] N-(1-méthyl 1H-indol -4-yl) benzami de et son chlorhydrate.

**13.** Procédé selon la revendication 2 ou 3, caractérisé en ce que l'on utilise à titre de principe actif le 2-[2-hydroxy 3-[(1,1,3,3-tétraméthylbutylamino] propoxy] N-(1H-indol-4-yl) benzamide.

**14.** Procédé selon la revendication 2 ou 3, caractérisé en ce que l'on utilise à titre de principe actif le 2-[2-hydroxy 3-[4-(diphénylméthyl) 1-pipérazinyl] propoxy] N-(1H-indol-4-yl) benzamide et son oxalate neutre.

**Claims**

**Claims for the following Contracting States : AT, BE, CH, DE, FR, GB, IT, LI, LU, NL, SE**

**1.** New compounds of formula (I):

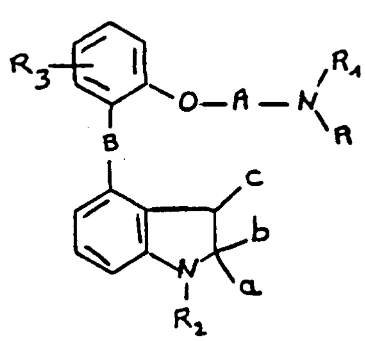

(I)

in which

- either $R_2$ and $R_3$ each represent a hydrogen atom, B represents a -CONH- chain, NH being on the indole side, b represents a hydrogen atom, a and c together form a second bond between the carbons which carry them, A represents a

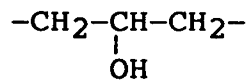

chain and either $R_1$ represents a hydrogen atom and R represents a radical chosen from the following radicals: 1,1-dimethylpropyl,

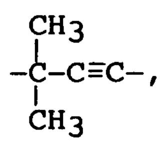,
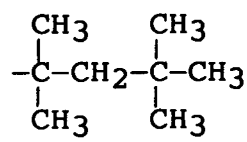

or

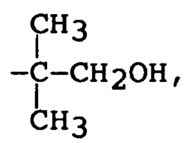

or R and $R_1$ form together with the nitrogen atom to which they are linked a morpholine nucleus,
- or $R_2$ and $R_3$ each represent a hydrogen atom, B represents a -CO-NH chain, NH being on the indole side, b represents a hydrogen atom and a and c together form a second bond between the carbons which carry them, A represents a $-(CH_2)_3-$ chain, $R_1$ represents a hydrogen atom and R a radical chosen from the following radicals: 1,1-dimethylpropyl, cyclohexyl, cyclohexylmethyl, propyl, isopropyl or

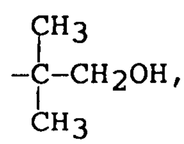

- or $R_2$ and $R_3$ each represent a hydrogen atom, B represents -NH-CO,

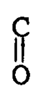

being on the indole side, b represents a hydrogen atom and a and c together form a second bond between the carbons which carry them, A represents the $-(CH_2)_3-$ chain, $R_1$ represents a hydrogen atom and R represents a radical chosen from the following radicals: cyclopentyl, cyclohexyl, 1,1-dimethylpropyl or

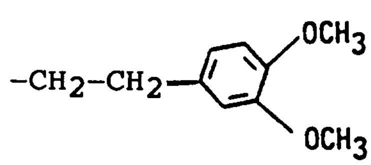

- or $R_2$ and $R_3$ represent a hydrogen atom, B represents -NH-CO-, -NH being on the indole side, b represents a hydrogen atom and a and c together form a second bond between the carbons

which carry them, A represents the $-(CH_2)_4$-chain, $R_1$ represents a hydrogen atom and R represents the 1,1-dimethylethyl group,

- or $R_2$ represents a methyl radical and $R_3$ represents a hydrogen atom, B represents -NH-CO-, NH being on the indole side, b represents a hydrogen atom and a and c together form a second bond between the carbons which carry them, A represents the

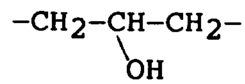

chain and R represents the 1,1-dimethylethyl group,

- or the $R_1$ and R substituents form with the nitrogen atom to which they are attached a

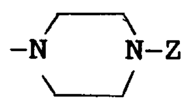

group in which Z represents a

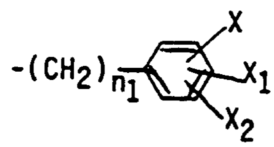

group or a

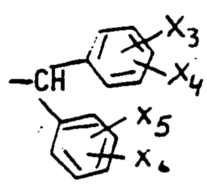

group, in which $n_1$ can take the values 1, 2 or 3 and X, $X_1$, $X_2$, $X_3$, $X_4$, $X_5$ and $X_5$, identical or different, represent a hydrogen atom, an alkyl radical containing 1 to 5 carbon atoms, an alkoxy radical containing 1 to 3 carbon atoms, a halogen atom, a nitro, amino, monoalkylamino or dialkylamino radical, on condition that all three of X, $X_1$ and $X_2$ do not represent a hydrogen atom, A represents a $-(CH_2)_n-$ chain in which n can take the values 2, 3, 4 or 5 or a

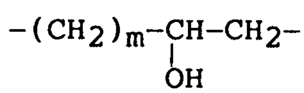

chain in which m can take the values 1, 2 or 3, B represents a -CO-NH-or -NH-CO- chain, $R_3$ represents a hydrogen atom, an alkyl radical containing 1 to 5 carbon atoms, an alkoxy radical containing 1 to 3 carbon atoms, a chlorine, bromine or iodine atom, a nitro radical or an amino radical optionally substituted by an aliphatic acyl group containing 2 to 5 carbon atoms or by an alkyl radical containing 1 to 5 carbon atoms, a represents together with b an oxo function, or represents together with c a second bond between the carbons which carry them, b represents a hydrogen atom, or together with a an oxo function, c represents a hydrogen atom, or together with a represents a second bond between the carbons which carry them, $R_2$ represents a hydrogen atom, a linear alkyl radical containing 1 to 5 carbon atoms or a branched alkyl radical containing 3 to 5 carbon atoms or an alkenyl or alkynyl radical containing 2 to 5 carbon atoms or

an aralkyl radical containing 7 to 12 carbon atoms optionally substituted by 1, 2 or 3 radicals chosen from the group constituted by halogen atoms and the following radicals: methyl, ethyl, methoxy, ethoxy, trifluoromethyl, methylthio, amino and nitro, or a cycloalkylalkyl radical containing 4 to 7 carbon atoms, as well as their addition salts with mineral or organic acids.

**2.** Compounds of formula (I), as defined in claim 1, in which the $R_1$ and R substituents form with the nitrogen atom to which they are linked a

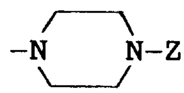

group in which Z has the previous meaning, A represents a

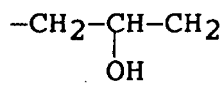

or $-(CH_2)_3$-or $-(CH_2)_4$- chain, $R_3$ represents a hydrogen atom, $R_2$ represents a hydrogen atom or a methyl radical, b represents a hydrogen atom and a and c form together a second bond between the carbons which carry them, as well as their addition salts with mineral or organic acids.

**3.** 2-[3-[(1,1-dimethylpropyl) amino] 2-hydroxypropoxy] N-(1H-indol-4-yl) benzamide and its benzoate and neutral oxalate.

**4.** 2-[3-[(1,1-dimethylethyl) amino] 2-hydroxypropoxy] N-(1-methyl 1H-indol-4-yl) benzamide and its hydrochloride.

**5.** 2-[2-hydroxy 3-[(1,1,3,3-tetramethylbutyl) amino] propoxy] N-(1H-indol-4-yl) benzamide, and 2-[2-hydroxy 3-[4-(diphenylmethyl) 1-piperazinyl] propoxy] N-(1H-indol-4-yl) benzamide and its neutral oxalate.

**6.** Preparation process for derivatives as defined by formula (I) of claim 1, as well as their salts, characterized in that a derivative of formula (II):

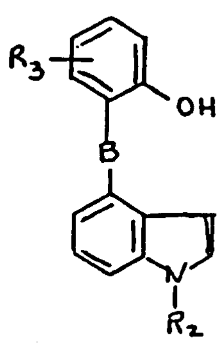

(II)

in which B and $R_3$ have the meaning already indicated and $R_2$ represents a hydrogen atom or a methyl, is reacted
either with a halide of formula (III):

Hal-G (III)

in which Hal represents a chlorine, bromine or iodine atom and -G represents a group of formula $-(CH_2)_n$-D, in which D represents a chlorine, bromine or iodine atom or a hydroxy radical or a sulphonate of this hydroxy radical, and n has the meaning already indicated, or -G represents a

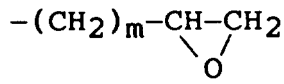

group in which m has the meaning already indicated, in order to obtain a derivative of formula (IV):

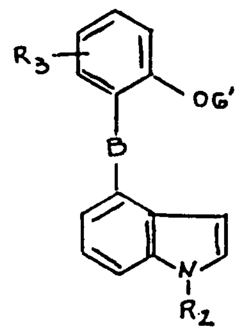

(IV)

in which B, $R_2$ and $R_3$ have the meaning already indicated, and G' represents a group of formula $-(CH_2)_n$-Hal in which n and Hal have the meaning already indicated, or a group of formula

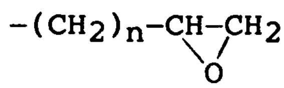

defined above, which is reacted with an amine of formula (V):

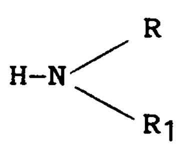

(V)

in which R and $R_1$ have the meaning already indicated, in order to obtain a product of formula ($I_A$):

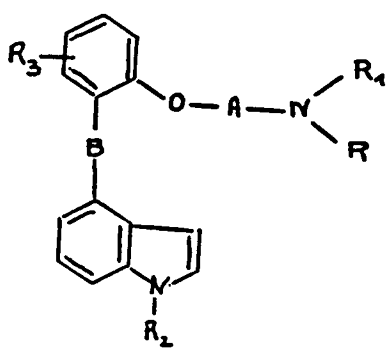

($I_A$)

in which A, B, R, $R_1$, $R_2$ and $R_3$ have the meaning already indicated, which either is isolated and, if desired, salified, or in the case where in the product of formula ($I_A$) R and $R_1$ form with the nitrogen atom to which they are linked a

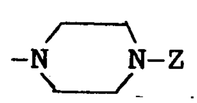

group in which Z has the meaning already indicated, the corresponding product of formula ($I_B$):

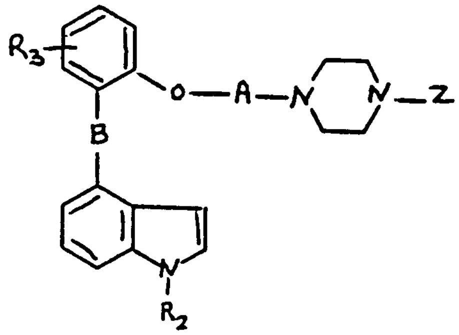

($I_B$)

in which A, B, $R_2$, $R_3$ and Z have the meanings already indicated, is subjected to the action of a halogenation agent in order to obtain a product of formula (VII):

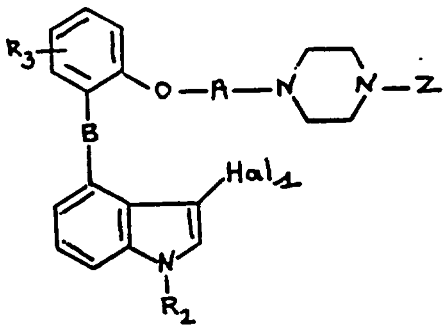

(VII)

in which A, B, $R_2$, $R_3$ and Z have the meanings already indicated and $Hal_1$ represents a bromine or chlorine atom, which is subjected to a hydrolysis in order to obtain a compound of formula ($I_C$):

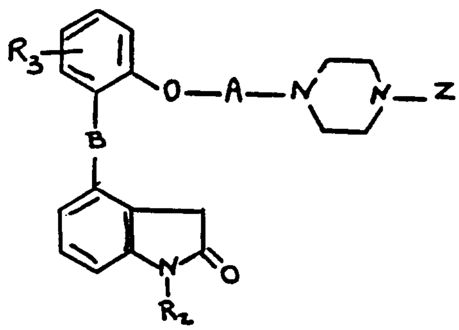

($I_C$)

in which A, B, $R_2$, $R_3$ and Z have the meanings already indicated, which is isolated and if desired salified, or the said product of formula (II) is reacted with a derivative of formula (VI):

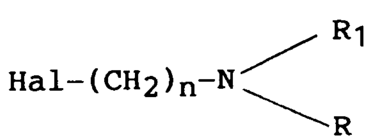

(VI)

in which Hal, n, R and $R_1$ have the meaning already indicated, in order to obtain the derivative of formula ($I_A$) in which A represents a $-(CH_2)_n$ chain, which is isolated and if desired salified, or in the case where in the product of formula ($I_A$) R and $R_1$ form with the nitrogen atom to which they are attached a

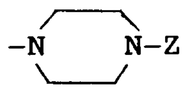

group in which Z has the meaning already indicated, the corresponding product of formula ($I_B$) is subjected to a halogenation agent then to a hydrolysis reagent in order to obtain the product of formula ($I_C$) which is isolated and if desired salified.

**7.** Medicaments, characterized in that they are constituted by new benzamide derivatives as defined by claim 1, as well as their addition salts with pharmaceutically acceptable acids.

**8.** Medicaments, characterized in that they are constituted by new benzamide derivatives as defined in any one of claims 2 to 5, as well as their addition salts with pharmaceutically acceptable acids.

**9.** Pharmaceutical compositions, characterized in that they contain, as active ingredient, at least one of the medicaments as defined in one of claims 7 or 8.

**10.** As new industrial compounds, the compounds of formula (VII) as defined in claim 6.

**Claims for the following Contracting State: ES**

**1.** Preparation process for new compounds of formula (I):

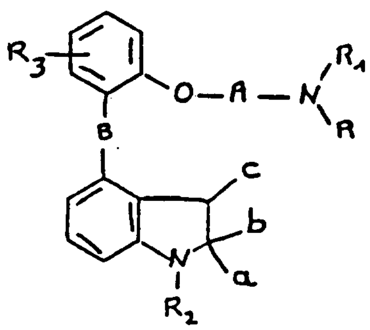

(I)

in which

- either $R_2$ and $R_3$ each represent a hydrogen atom, B represents a -CONH- chain, NH being on the indole side, b represents a hydrogen atom, a and c together form a second bond between the carbons which carry them, A represents a

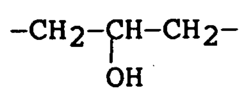

chain and either $R_1$ represents a hydrogen atom and R represents a radical chosen from the following radicals: 1,1-dimethylpropyl,

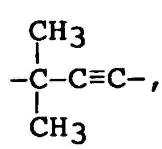

, 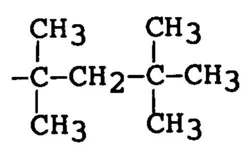

or

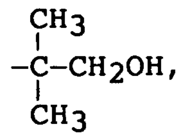

or R and $R_1$ form together with the nitrogen atom to which they are linked a morpholine nucleus,

- or $R_2$ and $R_3$ each represent a hydrogen atom, B represents a -CO-NH chain, NH being on the indole side, b represents a hydrogen atom and a and c together form a second bond between the carbons which carry them, A represents a $-(CH_2)_3-$ chain, $R_1$ represents a hydrogen atom and R a radical chosen from the following radicals: 1,1-dimethylpropyl, cyclohexyl, cyclohexylmethyl, propyl, isopropyl or

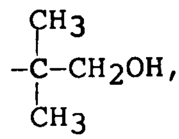

- or $R_2$ and $R_3$ each represent a hydrogen atom, B represents -NH-CO,

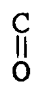

being on the indole side, b represents a hydrogen atom and a and c together form a second bond between the carbons which carry them, A represents the $-(CH_2)_3-$ chain, $R_1$ represents a hydrogen atom and R represents a radical chosen from the following radicals: cyclopentyl, cyclohexyl, 1,1-dimethylpropyl or

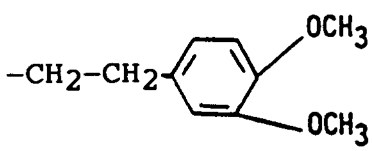

- or $R_2$ and $R_3$ represent a hydrogen atom, B represents -NH-CO-, -NH being on the indole side, b represents a hydrogen atom and a and c together form a second bond between the carbons which carry them, A represents the $-(CH_2)_4-$chain, $R_1$ represents a hydrogen atom and R represents the 1,1-dimethylethyl group,
- or $R_2$ represents a methyl radical and $R_3$ represents a hydrogen atom, B represents -NH-CO-, NH being on the indole side, b represents a hydrogen atom and a and c together form a second bond between the carbons which carry them, A represents the

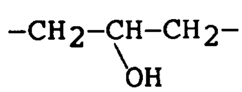

chain and R represents the 1,1-dimethylethyl group,

- or the $R_1$ and R substituents form with the nitrogen atom to which they are attached a

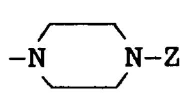

group in which Z represents a

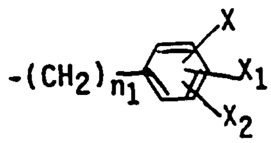

group or a

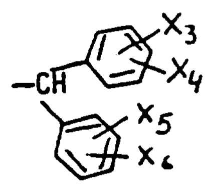

group, in which $n_1$ can take the values 1, 2 or 3 and X, $X_1$, $X_2$, $X_3$, $X_4$, $X_5$ and $X_5$, identical or different, represent a hydrogen atom, an alkyl radical containing 1 to 5 carbon atoms, an alkoxy radical containing 1 to 3 carbon atoms, a halogen atom, a nitro, amino, monoalkylamino or dialkylamino radical, on condition that all three of X, $X_1$ and $X_2$ do not represent a hydrogen atom, A represents a $-(CH_2)_n-$ chain in which n can take the values 2, 3, 4 or 5 or a

$$-(CH_2)_m-\underset{\displaystyle OH}{\underset{|}{C}H}-CH_2-$$

chain in which m can take the values 1, 2 or 3, B represents a -CO-NH-or -NH-CO- chain, $R_3$ represents a hydrogen atom, an alkyl radical containing 1 to 5 carbon atoms, an alkoxy radical containing 1 to 3 carbon atoms, a chlorine, bromine or iodine atom, a nitro radical or an amino radical optionally substituted by an aliphatic acyl group containing 2 to 5 carbon atoms or by an alkyl radical containing 1 to 5 carbon atoms, a represents together with b an oxo function, or represents together with c a second bond between the carbons which carry them, b represents a hydrogen atom, or together with a an oxo function, c represents a hydrogen atom, or together with a represents a second bond between the carbons which carry them, $R_2$ represents a hydrogen atom, a linear alkyl radical containing 1 to 5 carbon atoms or a branched alkyl radical containing 3 to 5 carbon atoms or an alkenyl or alkynyl radical containing 2 to 5 carbon atoms or an aralkyl radical containing 7 to 12 carbon atoms optionally substituted by 1, 2 or 3 radicals chosen from the group constituted by halogen atoms and the following radicals: methyl, ethyl, methoxy, ethoxy, trifluoromethyl, methylthio, amino and nitro, or a cycloalkylalkyl radical containing 4 to 7 carbon atoms, as well as their addition salts with mineral or organic acids, characterized in that a derivative of formula (II):

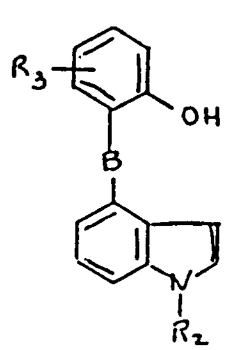

(II)

in which B and $R_3$ have the meaning already indicated and $R_2$ represents a hydrogen atom or a methyl, is reacted

either with a halide of formula (III):

Hal-G (III)

in which Hal represents a chlorine, bromine or iodine atom and -G represents a group of formula $-(CH_2)_n-D$, in which D represents a chlorine, bromine or iodine atom or a hydroxy radical or a sulphonate of this hydroxy radical, and n has the meaning already indicated, or -G represents a

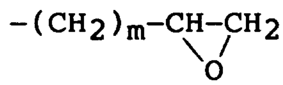

group in which m has the meaning already indicated, in order to obtain a derivative of formula (IV):

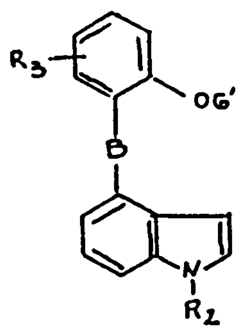

(IV)

in which B, $R_2$ and $R_3$ have the meaning already indicated, and G' represents a group of formula $-(CH_2)_n-Hal$ in which n and Hal have the meaning already indicated, or a group of formula

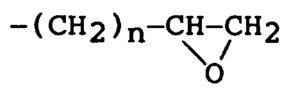

defined above, which is reacted with an amine of formula (V):

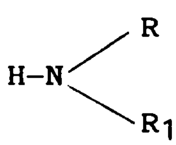

(V)

in which R and $R_1$ have the meaning already indicated, in order to obtain a product of formula $(I_A)$:

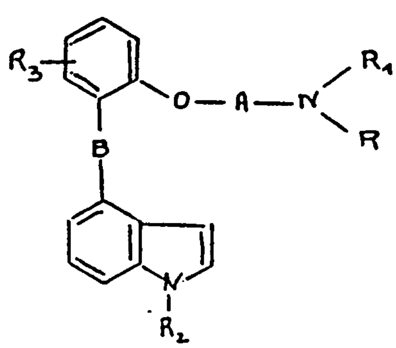

$(I_A)$

in which A, B, R, $R_1$, $R_2$ and $R_3$ have the meaning already indicated, which either is isolated and, if desired, salified, or in the case where in the product of formula ($I_A$) R and $R_1$ form with the nitrogen atom to which they are linked a

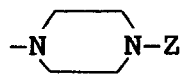

group in which Z has the meaning already indicated, the corresponding product of formula ($I_B$):

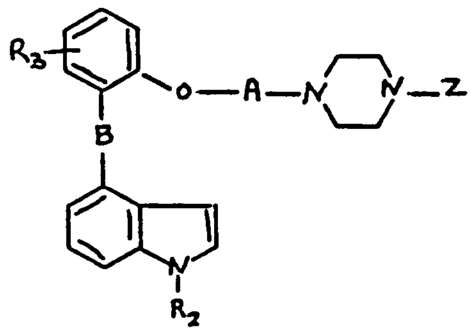

($I_B$)

in which A, B, $R_2$, $R_3$ and Z have the meanings already indicated, is subjected to the action of a halogenation agent in order to obtain a product of formula (VII):

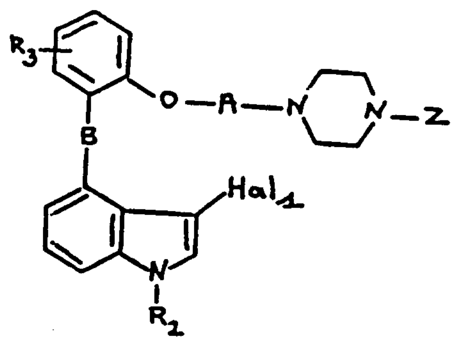

(VII)

in which A, B, $R_2$, $R_3$ and Z have the meanings already indicated and $Hal_1$ represents a bromine or chlorine atom, which is subjected to a hydrolysis in order to obtain a compound of formula ($I_C$):

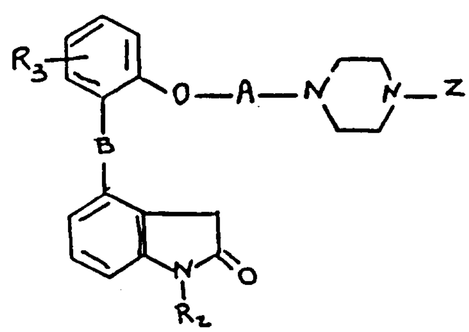

($I_C$)

in which A, B, $R_2$, $R_3$ and Z have the meanings already indicated, which is isolated and if desired salified, or the said product of formula (II) is reacted with a derivative of formula (VI):

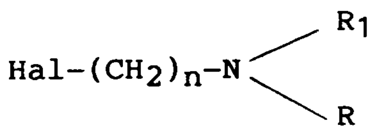

(VI)

in which Hal, n, R and $R_1$ have the meaning already indicated, in order to obtain the derivative of formula ($I_A$) in which A represents a $-(CH_2)n$ chain, which is isolated and if desired salified, or in the case where in the product of formula ($I_A$) R and $R_1$ form with the nitrogen atom to which they are attached a

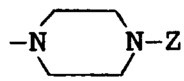

group in which Z has the meaning already indicated, the corresponding product of formula ($I_B$) is subjected to a halogenation agent then to a hydrolysis reagent in order to obtain the product of formula ($I_C$) which is isolated and if desired salified.

**2.** Process according to claim 1, characterized in that a product of formula (II) is used at the start in which $R_3$ represents a hydrogen atom, $R_2$ represents a hydrogen atom or a methyl radical, which is subjected to a halide of formula (III) Hal-G in which G represents a $-(CH_2)_nD$ group in which n is 2 or 3 or a

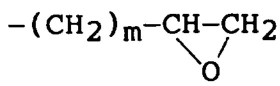

group in which m is 1, and a product of formula (V) or (VI) in which $R_1$ and R form with the nitrogen atom to which they are linked a

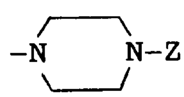

group in which Z has the meaning indicated in claim 1.

**Claims for the following Contracting State: GR**

**1.** Preparation process for new compounds of formula (I):

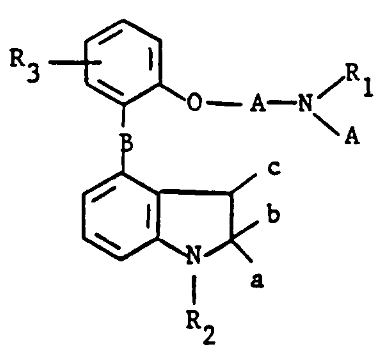

(I)

in which

- either $R_2$ and $R_3$ each represent a hydrogen atom, B represents a -CONH- chain, NH being on the indole side, b represents a hydrogen atom, a and c together form a second bond between the carbons which carry them, A represents a

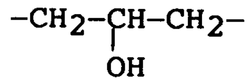

chain and either $R_1$ represents a hydrogen atom and R represents a radical chosen from the following radicals: 1,1-dimethylpropyl,

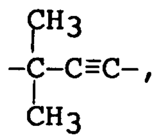

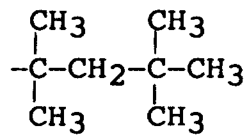

or

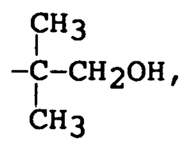

or R and $R_1$ form together with the nitrogen atom to which they are linked a morpholine nucleus,

- or $R_2$ and $R_3$ each represent a hydrogen atom, B represents a -CO-NH chain, NH being on the indole side, b represents a hydrogen atom and a and c together form a second bond between the carbons which carry them, A represents a $-(CH_2)_3-$ chain, $R_1$ represents a hydrogen atom and R a radical chosen from the following radicals: 1,1-dimethylpropyl, cyclohexyl, cyclohexylmethyl, propyl, isopropyl or

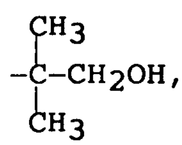

- or $R_2$ and $R_3$ each represent a hydrogen atom, B represents -NH-CO,

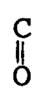

being on the indole side, b represents a hydrogen atom and a and c together form a second bond between the carbons which carry them, A represents the $-(CH_2)_3-$ chain, $R_1$ represents a hydrogen atom and R represents a radical chosen from the following radicals: cyclopentyl, cyclohexyl, 1,1-dimethylpropyl or

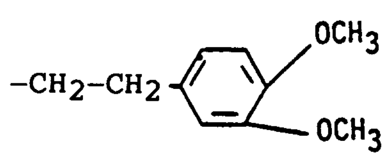

- or $R_2$ and $R_3$ represent a hydrogen atom, B represents -NH-CO-, -NH being on the indole side, b represents a hydrogen atom and a and c together form a second bond between the carbons which carry them, A represents the $-(CH_2)_4$-chain, $R_1$ represents a hydrogen atom and R represents the 1,1-dimethylethyl group,
- or $R_2$ represents a methyl radical and $R_3$ represents a hydrogen atom, B represents -NH-CO-, NH being on the indole side, b represents a hydrogen atom and a and c together form a second bond between the carbons which carry them, A represents the

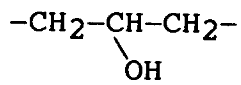

chain and R represents the 1,1-dimethylethyl group,
- or the $R_1$ and R substituents form with the nitrogen atom to which they are attached a

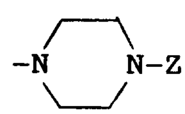

group in which Z represents a

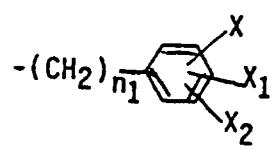

group or a

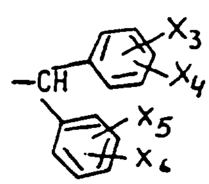

group, in which $n_1$ can take the values 1, 2 or 3 and X, $X_1$, $X_2$, $X_3$, $X_4$, $X_5$ and $X_5$, identical or different, represent a hydrogen atom, an alkyl radical containing 1 to 5 carbon atoms, an alkoxy radical containing 1 to 3 carbon atoms, a halogen atom, a nitro, amino, monoalkylamino or dialkylamino radical, on condition that all three of X, $X_1$ and $X_2$ do not represent a hydrogen atom, A represents a $-(CH_2)_n-$ chain in which n can take the values 2, 3, 4 or 5 or a

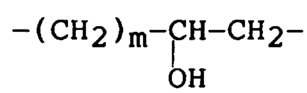

chain in which m can take the values 1, 2 or 3, B represents a -CO-NH-or -NH-CO- chain, $R_3$ represents a hydrogen atom, an alkyl radical containing 1 to 5 carbon atoms, an alkoxy radical containing 1 to 3 carbon atoms, a chlorine, bromine or iodine atom, a nitro radical or an amino radical optionally substituted by an aliphatic acyl group containing 2 to 5 carbon atoms or by an alkyl radical containing 1 to 5 carbon atoms, a represents together with b an oxo function, or represents together with c a second bond between the carbons which carry them, b represents a hydrogen atom, or together with a an oxo function, c represents a hydrogen atom, or together with a represents a second bond between the carbons which carry them, $R_2$ represents a hydrogen atom, a linear alkyl radical containing 1 to 5 carbon atoms or a branched alkyl radical containing 3 to 5 carbon atoms or an alkenyl or alkynyl radical containing 2 to 5 carbon atoms or

an aralkyl radical containing 7 to 12 carbon atoms optionally substituted by 1, 2 or 3 radicals chosen from the group constituted by halogen atoms and the following radicals: methyl, ethyl, methoxy, ethoxy, trifluoromethyl, methylthio, amino and nitro, or a cycloalkylalkyl radical containing 4 to 7 carbon atoms, as well as their addition salts with mineral or organic acids, characterized in that a derivative of formula (II):

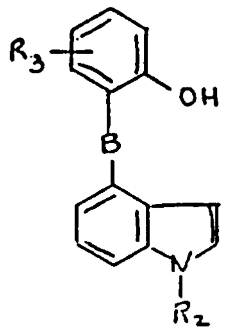

(II)

in which B and $R_3$ have the meaning already indicated and $R_2$ represents a hydrogen atom or a methyl, is reacted
either with a halide of formula (III):

Hal-G (III)

in which Hal represents a chlorine, bromine or iodine atom and -G represents a group of formula $-(CH_2)_n-D$, in which D represents a chlorine, bromine or iodine atom or a hydroxy radical or a sulphonate of this hydroxy radical, and n has the meaning already indicated, or -G represents a

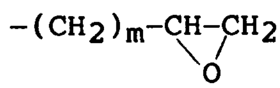

group in which m has the meaning already indicated, in order to obtain a derivative of formula (IV):

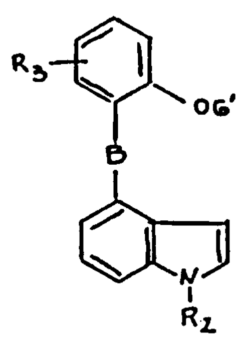

(IV)

in which B, $R_2$ and $R_3$ have the meaning already indicated, and G, represents a group of formula $-(CH_2)_n-Hal$ in which n and Hal have the meaning already indicated, or a group of formula

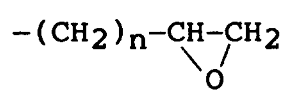

defined above, which is reacted with an amine of formula (V):

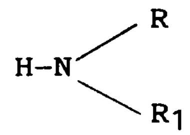

(V)

in which R and $R_1$ have the meaning already indicated, in order to obtain a product of formula ($I_A$):

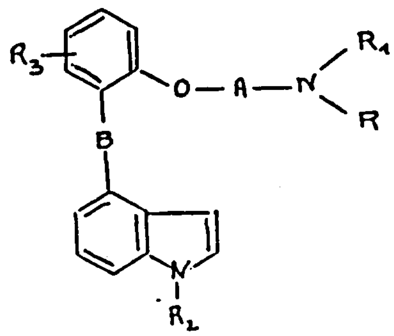

($I_A$)

in which A, B, R, $R_1$, $R_2$ and $R_3$ have the meaning already indicated, which either is isolated and, if desired, salified, or in the case where in the product of formula ($I_A$) R and $R_1$ form with the nitrogen atom to which they are linked a

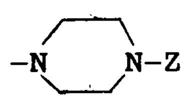

group in which Z has the meaning already indicated, the corresponding product of formula ($I_B$):

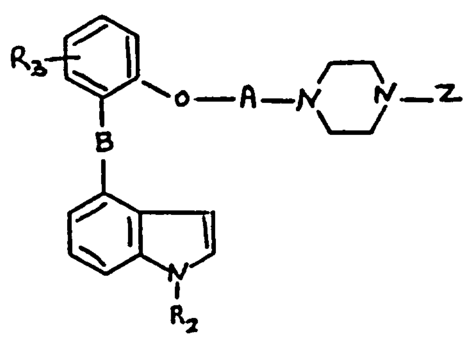

($I_B$)

in which A, B, $R_2$, $R_3$ and Z have the meanings already indicated, is subjected to the action of a halogenation agent in order to obtain a product of formula (VII):

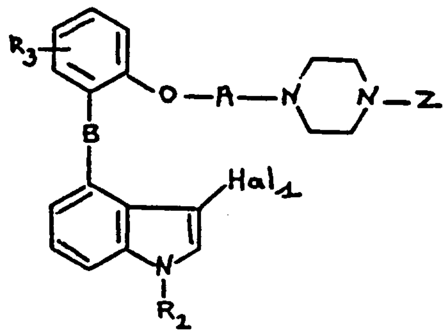

(VII)

in which A, B, $R_2$, $R_3$ and Z have the meanings already indicated and $Hal_1$ represents a bromine or chlorine atom, which is subjected to a hydrolysis in order to obtain a compound of formula ($I_C$):

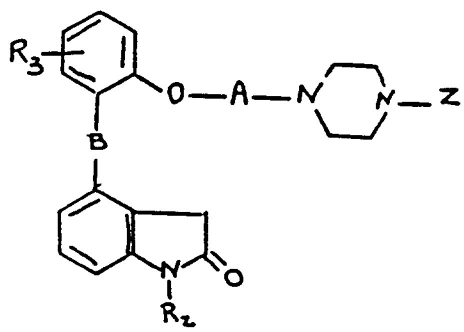

($I_C$)

in which A, B, $R_2$, $R_3$ and Z have the meanings already indicated, which is isolated and if desired salified, or the said product of formula (II) is reacted with a derivative of formula (VI):

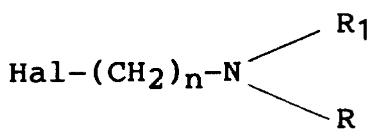

(VI)

in which Hal, n, R and $R_1$ have the meaning already indicated, in order to obtain the derivative of formula ($I_A$) in which A represents a $-(CH_2)_n$ chain, which is isolated and if desired salified, or in the case where in the product of formula ($I_A$) R and $R_1$ form with the nitrogen atom to which they are attached a

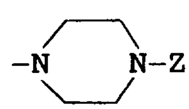

group in which Z has the meaning already indicated, the corresponding product of formula ($I_B$) is subjected to a halogenation agent then to a hydrolysis reagent in order to obtain the product of formula ($I_C$) which is isolated and if desired salified.

**2.** Process according to claim 1, characterized in that a product of formula (II) is used at the start in which $R_3$ represents a hydrogen atom, $R_2$ represents a hydrogen atom or a methyl radical, which is subjected to a halide of formula (III) Hal-G in which G represents a $-(CH_2)_nD$ group in which n is 2 or 3 or a

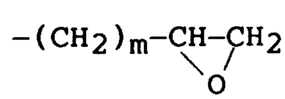

group in which m is 1, and a product of formula (V) or (VI) in which $R_1$ and R form with the nitrogen atom to which they are linked a

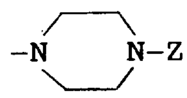

group in which Z has the meaning indicated in claim 1.

**3.** Process according to claim 1 or 2, characterized in that a product of formula (I) is prepared in which the $R_1$ and R substituents form with the nitrogen atom to which they are linked a

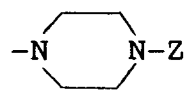

group in which Z has the previous meaning, A represents a

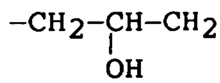

or $-(CH_2)_3-$ or $-(CH_2)_4-$ chain, $R_3$ represents a hydrogen atom, $R_2$ represents a hydrogen atom or a methyl radical, b represents a hydrogen atom and a and c together form a second bond between the carbons which carry them, as well as their addition salts with mineral or organic acids.

**4.** Process according to claim 1, characterized in that 2-[3-[(1,1-dimethylpropyl) amino] 2-hydroxypropoxy] N-(1H-indol-4-yl) benzamide and its benzoate and neutral oxalate are prepared.

**5.** Process according to claim 1, characterized in that 2-[3-[(1,1-dimethylethyl) amino] 2-hydroxypropoxy] N-(1-methyl 1H-indol-4-yl) benzamide and its hydrochloride are prepared.

**6.** Process according to claim 1, characterized in that 2-[2-hydroxy 3-[(1,1,3,3-tetramethylbutylamino] propoxy] N-(1H-indol-4-yl) benzamide is prepared.

**7.** Process according to claim 1, characterized in that 2-[2-hydroxy 3-[4-(diphenylmethyl) 1-piperazinyl] propoxy] N-(1H-indol-4-yl) benzamide and its neutral oxalate are prepared.

**8.** As new industrial compounds, the compounds of formula (VII) as defined in claim 1.

**9.** Preparation process for pharmaceutical compositions, characterized in that at least one of the products of formula (I) as defined in claim 1, or at least one of their addition salts with pharmaceutically acceptable acids, is used as active ingredient in a form intended for this use.

**10.** Preparation process for pharmaceutical compositions, characterized in that at least one of the products of formula (I) as defined in claim 2 or 3, or at least one of their addition salts with pharmaceutically acceptable acids, is used as active ingredient in a form intended for this use.

**11.** Process according to claim 4, characterized in that 2-[3-[(1,1-dimethylethyl) amino] 2-hydroxy propoxy] N-(1H-indol-4-yl) benzamide and its benzoate and neutral oxalate are used as active ingredient.

**12.** Process according to claim 2 or 3, characterized in that 2-[3-[(1,1-dimethylethyl) amino] 2-hydroxypropoxy] N-(1-methyl 1H-indol-4-yl) benzamide and its hydrochloride are used as active ingredient.

**13.** Process according to claim 2 or 3, characterized in that 2-[2-hydroxy 3[(1,1,3,3-tetramethylbutyl) amino] propoxy] N-(1H-indol-4-yl) benzamide is used as active ingredient.

**14.** Process according to claim 2 or 3, characterized in that 2-[2-hydroxy 3-[4-(diphenylmethyl) 1-piperazinyl] propoxy] N-(1H-indol-4-yl) benzamide and its neutral oxalate are used as active ingredient.

**Patentansprüche**
**Patentansprüche für folgende Vertragsstaaten : AT, BE, CH, DE, FR, GB, IT, LI, LU, NL, SE**

**1.** Neue Verbindungen der Formel (I)

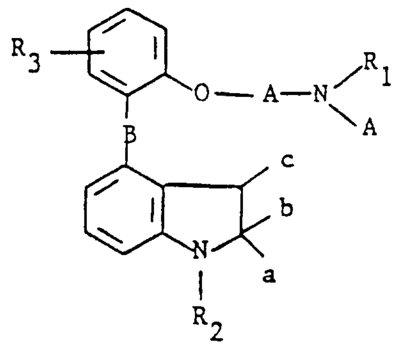

(I)

worin

- entweder $R_2$ und $R_3$ jeweils ein Wasserstoffatom bedeuten, B für eine Kette -CONH- steht, wobei sich NH neben dem Indol befindet, b ein Wasserstoffatom bedeutet, a und c gemeinsam zwischen den sie tragenden Kohlenstoffen eine zweite Bindung bilden, A eine Kette

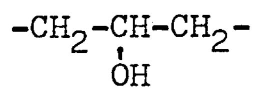

wiedergibt, und entweder $R_1$ ein Wasserstoffatom darstellt und R einen Rest, ausgewählt unter dem 1,1-Dimethylpropylrest und den Resten

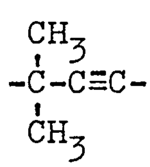 , 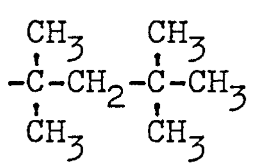 oder 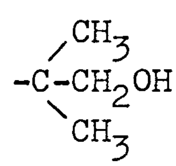 ,

bedeutet, oder R und $R_1$ gemeinsam mit dem Stickstoffatom, an das sie gebunden sind, einen Morpholinring bilden,
- oder $R_2$ und $R_3$ jeweils ein Wasserstoffatom bedeuten, B für eine Kette -CO-NH steht, wobei NH sich neben dem Indol befindet, b ein Wasserstoffatom bedeutet und a und c gemeinsam zwischen den sie tragenden Kohlenstoffen eine zweite Bindung bilden, A eine Kette $-(CH_2)_3$ - wiedergibt, $R_1$ ein Wasserstoffatom bedeutet und R einen Rest, ausgewählt unter den 1,1-Dimethylpropyl-, Cyclohexyl-, Cyclohexylmethyl-, Propyl-, Isopropylresten oder dem Rest

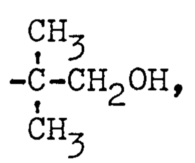

darstellt,

- oder $R_2$ und $R_3$ jeweils ein Wasserstoffatom bedeuten, B für -NH-CO steht, wobei

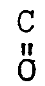

sich neben dem Indol befindet, b ein Wasserstoffatom bedeutet und a und c gemeinsam zwischen den sie tragenden Kohlenstoffen eine zweite Bindung bilden, A für die Kette $-(CH_2)_3-$ steht, $R_1$ ein Wasserstoffatom bedeutet und R einen Rest, ausgewählt unter den Cyclopentyl-, Cyclohexyl-, 1,1-Dimethylpropylresten oder dem Rest

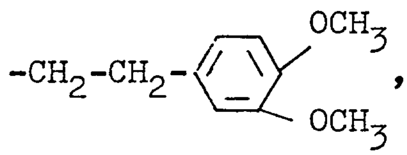

wiedergibt,
- oder $R_2$ und $R_3$ ein Wasserstoffatom bedeuten, B für -NH-CO-steht, wobei -NH sich neben dem Indol befindet, b ein Wasserstoffatom wiedergibt und a und c gemeinsam zwischen den sie tragenden Kohlenstoffen eine zweite Bindung bilden, A für die Kette $-(CH_2)_4-$ steht, $R_1$ ein Wasserstoffatom bedeutet und R die 1,1-Dimethylethylgruppe wiedergibt,
- oder $R_2$ eine Methylgruppe bedeutet und $R_3$ ein Wasserstoffatom wiedergibt, B für -NH-CO-steht, wobei NH sich neben dem Indol befindet, b ein Wasserstoffatom bedeutet und a und c gemeinsam zwischen den sie tragenden Kohlenstoffen eine zweite Bindung bilden, A für die Kette

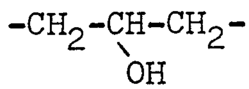

steht und R die 1,1-Dimethylethylgruppe bedeutet,
- oder die Substituenten $R_1$ und R mit dem Stickstoffatom, an das sie gebunden sind, eine Gruppe

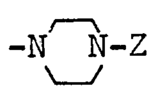

bilden, worin Z eine Gruppe

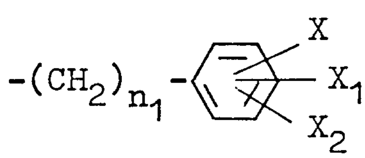

oder eine Gruppe

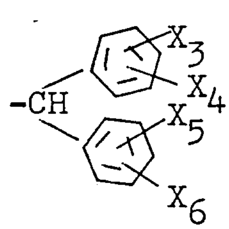

wiedergibt, worin $n_1$ die Werte 1, 2 oder 3 annehmen kann und X, $X_1$, $X_2$, $X_3$, $X_4$, $X_5$ und

$X_6$,gleich oder verschieden, ein Wasserstoffatom, einen Alkylrest mit 1 bis 5 Kohlenstoffatomen, einen Alkoxyrest mit 1 bis 3 Kohlenstoffatomen, ein Halogenatom, eine Nitro-, Amino-, Monoalkylamino- oder Dialkylaminogruppe bedeuten, mit der Maßgabe, daß X, $X_1$ und $X_2$ nicht alle drei ein Wasserstoffatom bedeuten, A für eine Kette $-(CH_2)_n-$, worin n die Werte 2, 3, 4 oder 5 annehmen kann, oder für eine Kette

$$-(CH_2)_m-\underset{OH}{CH}-CH_2-,$$

worin m die Werte 1, 2 oder 3 annehmen kann, steht, B eine Kette -CO-NH- oder -NH-CO- wiedergibt, $R_3$ ein Wasserstoffatom, eine Alkylgruppe mit 1 bis 5 Kohlenstoffatomen, eine Alkoxygruppe mit 1 bis 3 Kohlenstoffatomen, ein Chlor-, Brom- oder Jodatom, eine Nitrogruppe oder einen Aminorest, gegebenenfalls substituiert durch eine aliphatische Acylgruppe mit 2 bis 5 Kohlenstoffatomen oder durch einen Alkylrest mit 1 bis 5 Kohlenstoffatomen, bedeutet, a gemeinsam mit b eine Oxofunktion bildet oder gemeinsam mit c eine zweite Bindung zwischen den sie tragenden Kohlenstoffen wiedergibt, b ein Wasserstoffatom bedeutet oder gemeinsam mit a eine Oxofunktion bildet, c ein Wasserstoffatom bedeutet oder gemeinsam mit a eine zweite Bindung zwischen den sie tragenden Kohlenstoffen wiedergibt, $R_2$ ein Wasserstoffatom, einen linearen Alkylrest mit 1 bis 5 Kohlenstoffatomen oder einen verzweigten Alkylrest mit 3 bis 5 Kohlenstoffatomen oder einen Alkenyl- oder Alkinylrest mit 2 bis 5 Kohlenstoffatomen oder einen Aralkylrest mit 7 bis 12 Kohlenstoffatomen, gegebenenfalls substituiert durch 1, 2 oder 3 Reste, ausgewählt unter den Halogenatomen und den Methyl-, Ethyl-, Methoxy-, Ethoxy-, Trifluormethyl-, Methylthio-, Amino- und Nitrogruppen, oder einen Cycloalkalkylrest mit 4 bis 7 Kohlenstoffatomen bedeutet, sowie deren Additionssalze mit Mineral- oder organischen Säuren.

**2.** Verbindungen der Formel (I), wie in Anspruch 1 definiert, worin die Substituenten $R_1$ und R mit dem Stickstoffatom, an das sie gebunden sind, eine Gruppe

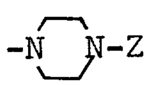

bilden, worin Z die vorstehende Bedeutung besitzt, A eine Kette

$$-CH_2-\underset{OH}{CH}-CH_2$$

oder $-(CH_2)_3-$ oder $-(CH_2)_4-$ wiedergibt, $R_3$ ein Wasserstoffatom bedeutet, $R_2$ ein Wasserstoffatom oder eine Methylgruppe wiedergibt, b für ein Wasserstoffatom steht und a und c gemeinsam zwischen den sie tragenden Kohlenstoffen eine zweite Bindung bilden, sowie deren Additionssalze mit Mineral- oder organischen Säuren.

**3.** 2-[3[(1,1-Dimethylpropyl)-amino]-2-hydroxypropoxy]-N-(1H-indol-4-yl)-benzamid und dessen Benzoat und neutrales Oxalat.

**4.** 2-[3-[(1,1-Dimethylethyl)-amino]-2-hydroxypropoxy]-N-(1-methyl-1H-indol-4-yl)-benzamid und dessen Hydrochlorid.

**5.** 2-[2-Hydroxy-3-[(1,1,3,3-tetramethylbutyl)-amino]-propoxy]-N-(1H-indol-4-yl)-benzamid und 2-[2-Hydroxy-3-[4-(diphenylmethyl)-1-piperazinyl]-propoxy]-N-(1H-indol-4-yl)-benzamid und dessen neutrales Oxalat.

**6.** Verfahren zur Herstellung der Derivate der Formel (I) gemäß Anspruch 1 sowie von deren Salzen, dadurch gekennzeichnet, daß man ein Derivat der Formel (II)

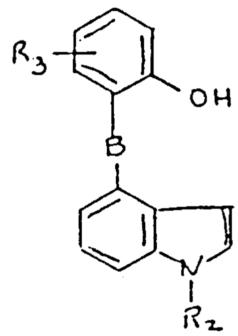

(II)

worin B und $R_3$ die vorstehend angegebene Bedeutung besitzen und $R_2$ für ein Wasserstoffatom oder eine Methylgruppe steht,
entweder mit einem Halogenid der Formel (III)

Hal-G (III)

worin Hal ein Chlor-, Brom- oder Jodatom bedeutet und -G für eine Gruppe der Formel $-(CH_2)_n$-D steht, worin D ein Chlor-, Brom- oder Jodatom oder eine Hydroxygruppe oder eine Sulfonatgruppe dieser Hydroxygruppe bedeutet und n die vorstehend angegebene Bedeutung besitzt, oder -G für eine Gruppe

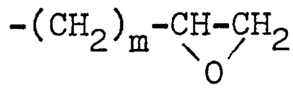

steht, worin m die vorstehende Bedeutung besitzt, umsetzt, um zu einem Derivat der Formel (IV)

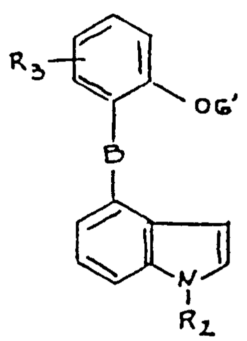

(IV)

zu gelangen, worin B, $R_2$ und $R_3$ die angegebene Bedeutung besitzen und G' eine Gruppe der Formel $-(CH_2)_n$-Hal, worin n und Hal die vorstehende Bedeutung besitzen, oder eine Gruppe der Formel

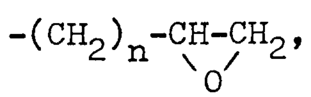

wie vorstehend definiert, wiedergibt, welches man mit einem Amin der Formel (V)

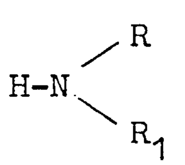

(V)

umsetzt, worin R und $R_1$ die angegebene Bedeutung besitzen, um zu einem Produkt der Formel ($I_A$)

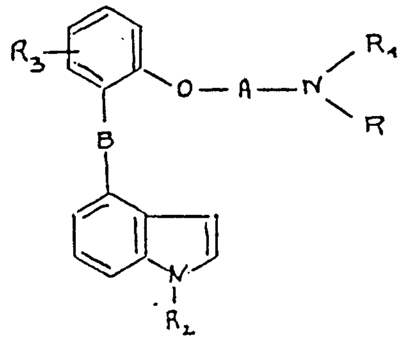

$(I_A)$

zu gelangen, worin A, B, R, $R_1$, $R_2$ und $R_3$ die angegebene Bedeutung besitzen, welches man entweder isoliert und gewünschtenfalls in ein Salz überführt oder für den Fall, daß in dem Produkt der Formel ($I_A$) R und $R_1$ mit dem Stickstoffatom, an das sie gebunden sind, eine Gruppe

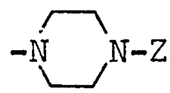

bilden, worin Z die vorstehend angegebene Bedeutung besitzt, man das entsprechende Produkt der Formel ($I_B$)

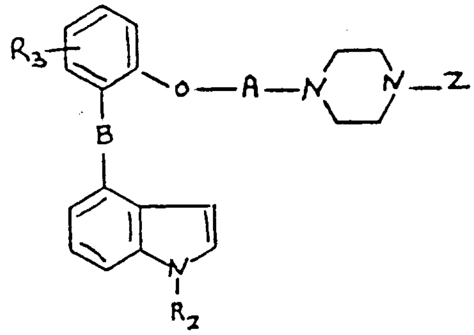

$(I_B)$

worin A, B, $R_2$, $R_3$ und Z die vorstehend angegebenenBedeutungen besitzen, der Einwirkung eines Halogenierungsmittels unterzieht, um zu einem Produkt der Formel (VII)

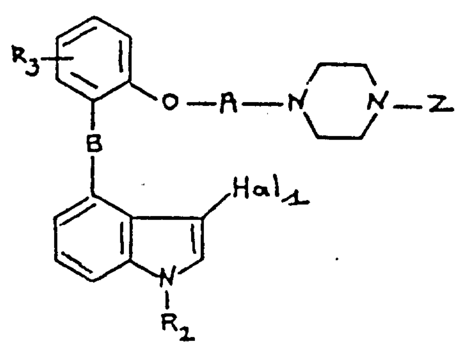

(VII)

zu gelangen, worin A, B, $R_2$, $R_3$ und Z die vorstehend angegebenen Bedeutungen besitzen und $Hal_1$ für ein Brom- oder Chloratom steht, welches man einer Hydrolyse unterzieht, um eine Verbindung der Formel ($I_C$)

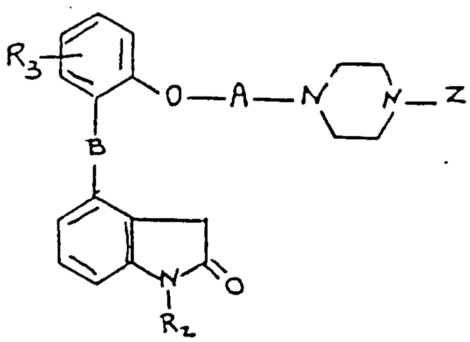

$(I_C)$

zu erhalten, worin A, B, $R_2$, $R_3$ und Z die angegebenen Bedeutungen besitzen, welches man isoliert und gewünschtenfalls in ein Salz überführt,
oder man das Produkt der Formel (II) mit einem Derivat der Formel (VI)

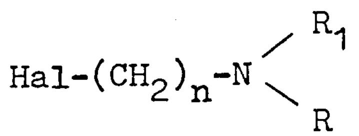

(VI)

umsetzt, worin Hal, n, R und $R_1$ die angegebene Bedeutung besitzen, um das Derivat der Formel ($I_A$) zu erhalten, worin A für eine Kette $-(CH_2)_n$ steht, welches man isoliert und gewünschtenfalls in ein Salz überführt, oder für den Fall, daß in dem Produkt der Formel ($I_A$) R und $R_1$ mit dem Stickstoffatom, an das sie gebunden sind, eine Gruppe

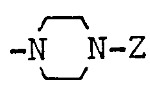

bilden, worin Z die angegebene Bedeutung besitzt, man das entsprechende Produkt der Formel ($I_B$) einem Halogenierungsmittel, dann einem Hydrolysereagens unterzieht, um das Produkt der Formel ($I_C$) zu erhalten, welches man isoliert und gewünschtenfalls in ein Salz überführt.

**7.** Arzneimittel, dadurch gekennzeichnet, daß sie aus den neuen Benzamid-Derivaten, wie in Anspruch 1 definiert, sowie aus deren Additionssalzen mit pharmazeutisch verträglichen Säuren bestehen.

**8.** Arzneimittel, dadurch gekennzeichnet, daß sie aus den neuen Benzamid-Derivaten, wie in einem der Ansprüche 2 bis 5 definiert, sowie aus deren Additionssalzen mit pharmazeutisch verträglichen Säuren bestehen.

**9.** Pharmazeutische Zusammensetzungen, dadurch gekennzeichnet, daß sie als Wirkstoff zumindest eines der Arzneimittel, wie in einem der Ansprüche 7 oder 8 definiert, enthalten.

**10.** Als neue industrielle Verbindungen die Verbindungen der Formel (VII), wie in Anspruch 6 definiert.

**Patentansprüch für folgenden Vertragsstaat : ES**

**1.** Verfahren zur Herstellung neuer Verbindungen der Formel (I)

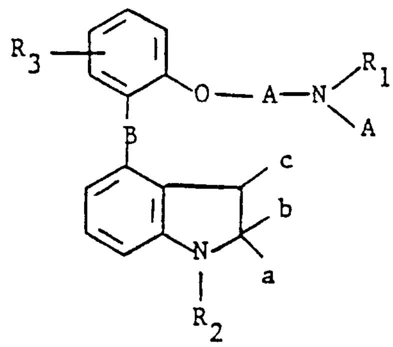

(I)

worin

- entweder $R_2$ und $R_3$ jeweils ein Wasserstoffatom bedeuten, B für eine Kette -CONH- steht, wobei sich NH neben dem Indol befindet, b ein Wasserstoffatom bedeutet, a und c gemeinsam zwischen den sie tragenden Kohlenstoffen eine zweite Bindung bilden, A eine Kette

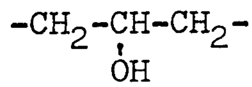

wiedergibt, und entweder $R_1$ ein Wasserstoffatom darstellt und R einen Rest, ausgewählt unter dem 1,1-Dimethylpropylrest und den Resten

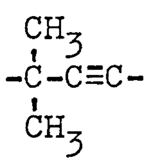 , 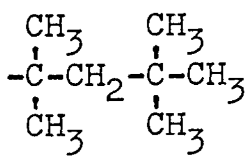 oder 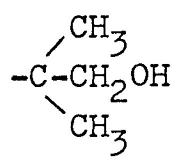 ,

bedeutet, oder R und $R_1$ gemeinsam mit dem Stickstoffatom, an das sie gebunden sind, einen Morpholinring bilden,

- oder $R_2$ und $R_3$ jeweils ein Wasserstoffatom bedeuten, B für eine Kette -CO-NH steht, wobei NH sich neben dem Indol befindet, b ein Wasserstoffatom bedeutet und a und c gemeinsam zwischen den sie tragenden Kohlenstoffen eine zweite Bindung bilden, A eine Kette $-(CH_2)_3-$ wiedergibt, $R_1$ ein Wasserstoffatom bedeutet und R einen Rest, ausgewählt unter den 1,1-Dimethylpropyl-, Cyclohexyl-, Cyclohexylmethyl-, Propyl-, Isopropylresten oder dem Rest

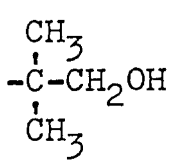 ,

darstellt,

- oder $R_2$ und $R_3$ jeweils ein Wasserstoffatom bedeuten, B für -NH-CO steht, wobei

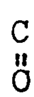

sich neben dem Indol befindet, b ein Wasserstoffatom bedeutet und a und c gemeinsam

zwischen den sie tragenden Kohlenstoffen eine zweite Bindung bilden, A für die Kette $-(CH_2)_3-$ steht, $R_1$ ein Wasserstoffatom bedeutet und R einen Rest, ausgewählt unter den Cyclopentyl-, Cyclohexyl-, 1,1-Dimethylpropylresten oder dem Rest

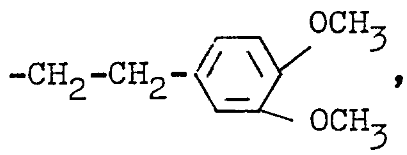

wiedergibt,

- oder $R_2$ und $R_3$ ein Wasserstoffatom bedeuten, B für -NH-CO-steht, wobei -NH sich neben dem Indol befindet, b ein Wasserstoffatom wiedergibt und a und c gemeinsam zwischen den sie tragenden Kohlenstoffen eine zweite Bindung bilden, A für die Kette $-(CH_2)_4-$ steht, $R_1$ ein Wasserstoffatom bedeutet und R die 1,1-Dimethylethylgruppe wiedergibt,
- oder $R_2$ eine Methylgruppe bedeutet und $R_3$ ein Wasserstoffatom wiedergibt, B für -NH-CO-steht, wobei NH sich neben dem Indol befindet, b ein Wasserstoffatom bedeutet und a und c gemeinsam zwischen den sie tragenden Kohlenstoffen eine zweite Bindung bilden, A für die Kette

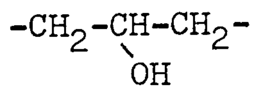

steht und R die 1,1-Dimethylethylgruppe bedeutet,

- oder die Substituenten $R_1$ und R mit dem Stickstoffatom, an das sie gebunden sind, eine Gruppe

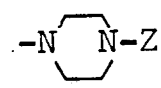

bilden, worin Z eine Gruppe

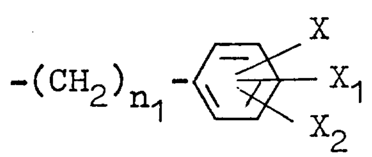

oder eine Gruppe

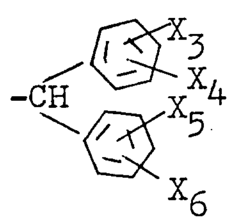

wiedergibt, worin $n_1$ die Werte 1, 2 oder 3 annehmen kann und X, $X_1$, $X_2$, $X_3$, $X_4$, $X_5$ und $X_5$,gleich oder verschieden, ein Wasserstoffatom, einen Alkylrest mit 1 bis 5 Kohlenstoffatomen, einen Alkoxyrest mit 1 bis 3 Kohlenstoffatomen, ein Halogenatom, eine Nitro-, Amino-, Monoalkylamino- oder Dialkylaminogruppe bedeuten, mit der Maßgabe, daß X, $X_1$ und $X_2$ nicht alle drei ein Wasserstoffatom bedeuten, A für eine Kette $-(CH_2)_n-$, worin n die Werte 2, 3, 4 oder 5 annehmen kann, oder für eine Kette

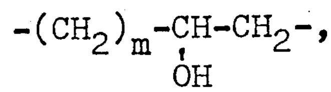

worin m die Werte 1, 2 oder 3 annehmen kann, steht, B eine Kette -CO-NH- oder -NH-CO- wiedergibt, $R_3$ ein Wasserstoffatom, eine Alkylgruppe mit 1 bis 5 Kohlenstoffatomen, eine Alkoxygruppe mit 1 bis 3 Kohlenstoffatomen, ein Chlor-, Brom- oder Jodatom, eine Nitrogruppe oder einen Aminorest, gegebenenfalls substituiert durch eine aliphatische Acylgruppe mit 2 bis 5 Kohlenstoffatomen oder durch einen Alkylrest mit 1 bis 5 Kohlenstoffatomen, bedeutet, a gemeinsam mit b eine Oxofunktion bildet oder gemeinsam mit c eine zweite Bindung zwischen den sie tragenden Kohlenstoffen wiedergibt, b ein Wasserstoffatom bedeutet oder gemeinsam mit a eine Oxofunktion bildet, c ein Wasserstoffatom bedeutet oder gemeinsam mit a eine zweite Bindung zwischen den sie tragenden Kohlenstoffen wiedergibt, $R_2$ ein Wasserstoffatom, einen linearen Alkylrest mit 1 bis 5 Kohlenstoffatomen oder einen verzweigten Alkylrest mit 3 bis 5 Kohlenstoffatomen oder einen Alkenyl- oder Alkinylrest mit 2 bis 5 Kohlenstoffatomen oder einen Aralkylrest mit 7 bis 12 Kohlenstoffatomen, gegebenenfalls substituiert durch 1, 2 oder 3 Reste, ausgewählt unter den Halogenatomen und den Methyl-, Ethyl-, Methoxy-, Ethoxy-, Trifluormethyl-, Methylthio-, Amino- und Nitrogruppen, oder einen Cycloalkalkylrest mit 4 bis 7 Kohlenstoffatomen bedeutet, sowie von deren Additionssalzen mit Mineral- oder organischen Säuren,
dadurch gekennzeichnet, daß man ein Derivat der Formel (II)

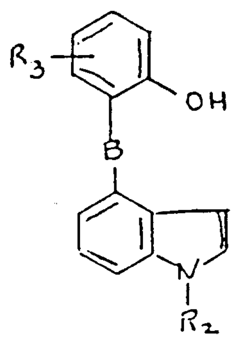

(II)

worin B und $R_3$ die vorstehend angegebene Bedeutung besitzen und $R_2$ für ein Wasserstoffatom oder eine Methylgruppe steht,
entweder mit einem Halogenid der Formel (III)

Hal-G (III)

worin Hal ein Chlor-, Brom- oder Jodatom bedeutet und -G für eine Gruppe der Formel $-(CH_2)_n-D$ steht, worin D ein Chlor-, Brom- oder Jodatom oder eine Hydroxygruppe oder eine Sulfonatgruppe dieser Hydroxygruppe bedeutet und n die vorstehend angegebene Bedeutung besitzt, oder -G für eine Gruppe

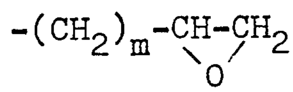

steht, worin m die vorstehende Bedeutung besitzt, umsetzt, um zu einem Derivat der Formel (IV)

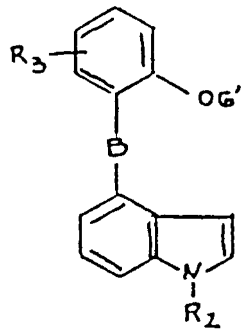

(IV)

zu gelangen, worin B, $R_2$ und $R_3$ die angegebene Bedeutung besitzen und G' eine Gruppe der Formel $-(CH_2)_n$-Hal, worin n und Hal die vorstehende Bedeutung besitzen, oder eine Gruppe der Formel

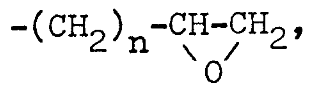

wie vorstehend definiert, wiedergibt, welches man mit einem Amin der Formel (V)

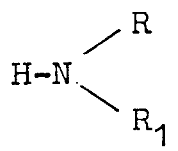

(V)

umsetzt, worin R und $R_1$ die angegebene Bedeutung besitzen, um zu einem Produkt der Formel ($I_A$)

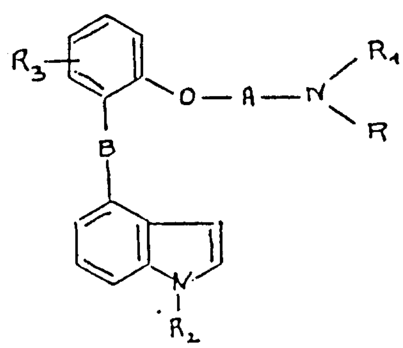

($I_A$)

zu gelangen, worin A, B, R, $R_1$, $R_2$ und $R_3$ die angegebene Bedeutung besitzen, welches man entweder isoliert und gewünschtenfalls in ein Salz überführt oder für den Fall, daß in dem Produkt der Formel ($I_A$) R und $R_1$ mit dem Stickstoffatom, an das sie gebunden sind, eine Gruppe

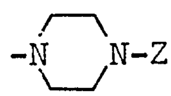

bilden, worin Z die vorstehend angegebene Bedeutung besitzt, man das entsprechende Produkt der Formel ($I_B$)

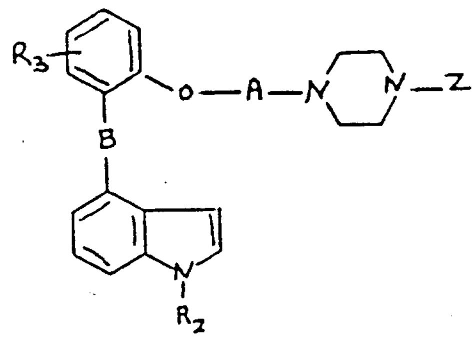

$(I_B)$

worin A, B, $R_2$, $R_3$ und Z die vorstehend angegebenenBedeutungen besitzen, der Einwirkung eines Halogenierungsmittels unterzieht, um zu einem Produkt der Formel (VII)

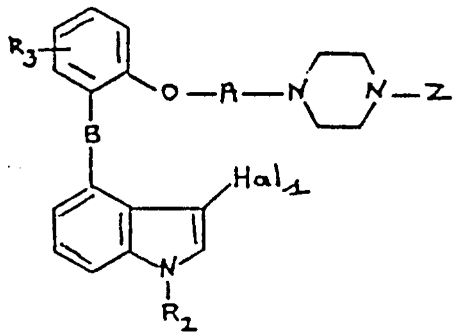

(VII)

zu gelangen, worin A, B, $R_2$, $R_3$ und Z die vorstehend angegebenen Bedeutungen besitzen und $Hal_1$ für ein Brom- oder Chloratom steht, welches man einer Hydrolyse unterzieht, um eine Verbindung der Formel ($I_C$)

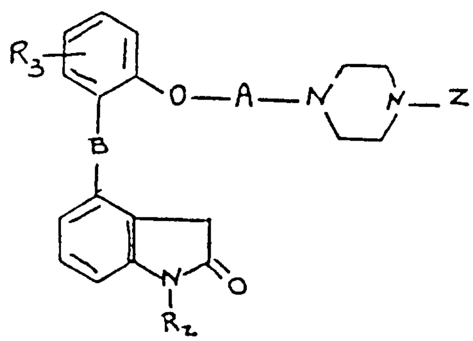

$(I_C)$

zu erhalten, worin A, B, $R_2$, $R_3$ und Z die angegebenen Bedeutungen besitzen, welches man isoliert und gewünschtenfalls in ein Salz überführt,
oder man das Produkt der Formel (II) mit einem Derivat der Formel (VI)

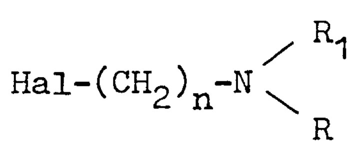

(VI)

umsetzt, worin Hal, n, R und $R_1$ die angegebene Bedeutung besitzen, um das Derivat der Formel ($I_A$) zu erhalten, worin A für eine Kette $-(CH_2)_n$ steht, welches man isoliert und gewünschtenfalls in ein Salz überführt, oder für den Fall, daß in dem Produkt der Formel ($I_A$) R und $R_1$ mit dem Stickstoffatom, an das sie gebunden sind, eine Gruppe

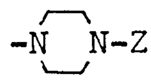

bilden, worin Z die angegebene Bedeutung besitzt, man das entsprechende Produkt der Formel ($I_B$) einem Halogenierungsmittel, dann einem Hydrolysereagens unterzieht, um das Produkt der Formel ($I_C$) zu erhalten, welches man isoliert und gewünschtenfalls in ein Salz überführt.

**2.** Verfahren gemäß Anspruch 1, dadurch gekennzeichnet, daß man von einem Produkt der Formel (II), worin $R_3$ ein Wasserstoffatom bedeutet, $R_2$ ein Wasserstoffatom oder einen Methylrest bedeutet, welches man mit einem Halogenid der Formel (III) Hal-G umsetzt, worin G eine Gruppe $-(CH_2)_nD$, wobei n für 2 oder 3 steht, oder eine Gruppe

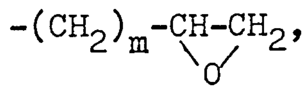

worin m für 1 steht, wiedergibt, und einem Produkt der Formel (V) oder (VI), worin $R_1$ und R mit dem Stickstoffatom, an, das sie gebunden sind, eine Gruppe

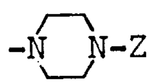

bilden, worin Z die in Anspruch 1 angegebene Bedeutung besitzt, ausgeht.

**Patentansprüche für folgenden Vertragsstaat : GR**

**1.** Verfahren zur Herstellung neuer Verbindungen der Formel (I)

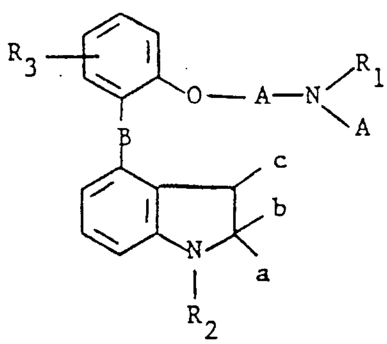

(I)

worin

- entweder $R_2$ und $R_3$ jeweils ein Wasserstoffatom bedeuten, B für eine Kette -CONH- steht, wobei sich NH neben dem Indol befindet, b ein Wasserstoffatom bedeutet, a und c gemeinsam zwischen den sie tragenden Kohlenstoffen eine zweite Bindung bilden, A eine Kette

$$-CH_2-\underset{OH}{CH}-CH_2-$$

wiedergibt, und entweder $R_1$ ein Wasserstoffatom darstellt und R einen Rest, ausgewählt unter dem 1,1-Dimethylpropylrest und den Resten

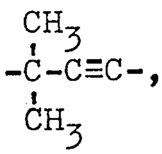, 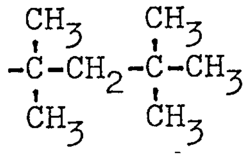 oder 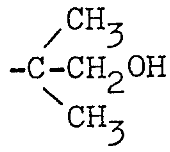 ,

bedeutet, oder R und $R_1$ gemeinsam mit dem Stickstoffatom, an das sie gebunden sind, einen Morpholinring bilden,

- oder $R_2$ und $R_3$ jeweils ein Wasserstoffatom bedeuten, B für eine Kette -CO-NH steht, wobei NH sich neben dem Indol befindet, b ein Wasserstoffatom bedeutet und a und c gemeinsam zwischen den sie tragenden Kohlenstoffen eine zweite Bindung bilden, A eine Kette $-(CH_2)_3-$ wiedergibt, $R_1$ ein Wasserstoffatom bedeutet und R einen Rest, ausgewählt unter den 1,1-Dimethylpropyl-, Cyclohexyl-, Cyclohexylmethyl-, Propyl-, Isopropylresten oder dem Rest

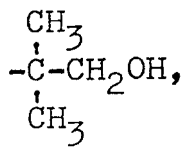

darstellt,

- oder $R_2$ und $R_3$ jeweils ein Wasserstoffatom bedeuten, B für -NH-CO steht, wobei

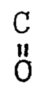

sich neben dem Indol befindet, b ein Wasserstoffatom bedeutet und a und c gemeinsam zwischen den sie tragenden Kohlenstoffen eine zweite Bindung bilden, A für die Kette $-(CH_2)_3-$ steht, $R_1$ ein Wasserstoffatom bedeutet und R einen Rest, ausgewählt unter den Cyclopentyl-, Cyclohexyl-, 1,1-Dimethylpropylresten oder dem Rest

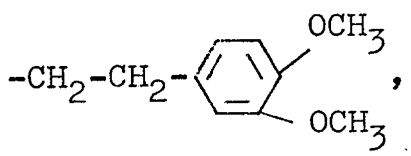

wiedergibt,

- oder $R_2$ und $R_3$ ein Wasserstoffatom bedeuten, B für -NH-CO-steht, wobei -NH sich neben dem Indol befindet, b ein Wasserstoffatom wiedergibt und a und c gemeinsam zwischen den sie tragenden Kohlenstoffen eine zweite Bindung bilden, A für die Kette $-(CH_2)_4-$ steht, $R_1$ ein Wasserstoffatom bedeutet und R die 1,1-Dimethylethylgruppe wiedergibt,
- oder $R_2$ eine Methylgruppe bedeutet und $R_3$ ein Wasserstoffatom wiedergibt, B für -NH-CO-steht, wobei NH sich neben dem Indol befindet, b ein Wasserstoffatom bedeutet und a und c gemeinsam zwischen den sie tragenden Kohlenstoffen eine zweite Bindung bilden, A für die Kette

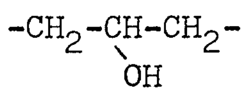

steht und R die 1,1-Dimethylethylgruppe bedeutet,

- oder die Substituenten $R_1$ und R mit dem Stickstoffatom, an das sie gebunden sind, eine Gruppe

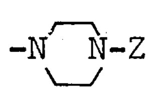

bilden, worin Z eine Gruppe

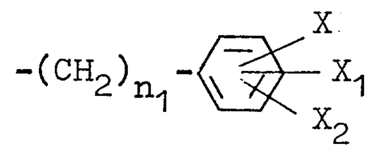

oder eine Gruppe

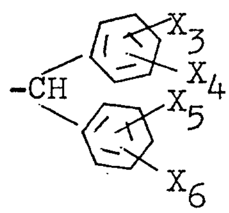

wiedergibt, worin $n_1$ die Werte 1, 2 oder 3 annehmen kann und X, $X_1$, $X_2$, $X_3$, $X_4$, $X_5$ und $X_5$,gleich oder verschieden, ein Wasserstoffatom, einen Alkylrest mit 1 bis 5 Kohlenstoffatomen, einen Alkoxyrest mit 1 bis 3 Kohlenstoffatomen, ein Halogenatom, eine Nitro-, Amino-, Monoalkylamino- oder Dialkylaminogruppe bedeuten, mit der Maßgabe, daß X, $X_1$ und $X_2$ nicht alle drei ein Wasserstoffatom bedeuten, A für eine Kette $-(CH_2)_n-$, worin n die Werte 2, 3, 4 oder 5 annehmen kann, oder für eine Kette

$$-(CH_2)_m-\underset{\displaystyle OH}{CH}-CH_2-,$$

worin m die Werte 1, 2 oder 3 annehmen kann, steht, B eine Kette -CO-NH- oder -NH-CO- wiedergibt, $R_3$ ein Wasserstoffatom, eine Alkylgruppe mit 1 bis 5 Kohlenstoffatomen, eine Alkoxygruppe mit 1 bis 3 Kohlenstoffatomen, ein Chlor-, Brom- oder Jodatom, eine Nitrogruppe oder einen Aminorest, gegebenenfalls substituiert durch eine aliphatische Acylgruppe mit 2 bis 5 Kohlenstoffatomen oder durch einen Alkylrest mit 1 bis 5 Kohlenstoffatomen, bedeutet, a gemeinsam mit b eine Oxofunktion bildet oder gemeinsam mit c eine zweite Bindung zwischen den sie tragenden Kohlenstoffen wiedergibt, b ein Wasserstoffatom bedeutet oder gemeinsam mit a eine Oxofunktion bildet, c ein Wasserstoffatom bedeutet oder gemeinsam mit a eine zweite Bindung zwischen den sie tragenden Kohlenstoffen wiedergibt, $R_2$ ein Wasserstoffatom, einen linearen Alkylrest mit 1 bis 5 Kohlenstoffatomen oder einen verzweigten Alkylrest mit 3 bis 5 Kohlenstoffatomen oder einen Alkenyl- oder Alkinylrest mit 2 bis 5 Kohlenstoffatomen oder einen Aralkylrest mit 7 bis 12 Kohlenstoffatomen, gegebenenfalls substituiert durch 1, 2 oder 3 Reste, ausgewählt unter den Halogenatomen und den Methyl-, Ethyl-, Methoxy-, Ethoxy-, Trifluormethyl-, Methylthio-, Amino- und Nitrogruppen, oder einen Cycloalkalkylrest mit 4 bis 7 Kohlenstoffatomen bedeutet, sowie von deren Additionssalzen mit Mineral- oder organischen Säuren,
dadurch gekennzeichnet, daß man ein Derivat der Formel (II)

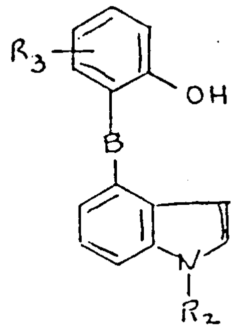

(II)

worin B und $R_3$ die vorstehend angegebene Bedeutung besitzen und $R_2$ für ein Wasserstoffatom oder eine Methylgruppe steht,
entweder mit einem Halogenid der Formel (III)

Hal-G (III)

worin Hal ein Chlor-, Brom- oder Jodatom bedeutet und -G für eine Gruppe der Formel $-(CH_2)_n-D$ steht, worin D ein Chlor-, Brom- oder Jodatom oder eine Hydroxygruppe oder eine Sulfonatgruppe dieser Hydroxygruppe bedeutet und n die vorstehend angegebene Bedeutung besitzt, oder -G für eine Gruppe

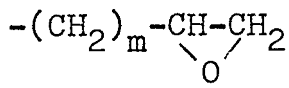

steht, worin m die vorstehende Bedeutung besitzt, umsetzt, um zu einem Derivat der Formel (IV)

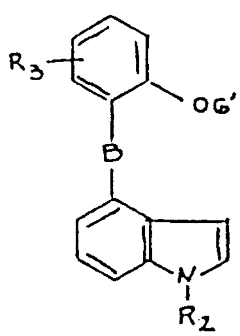

(IV)

zu gelangen, worin B, $R_2$ und $R_3$ die angegebene Bedeutung besitzen und G' eine Gruppe der Formel $-(CH_2)_n$-Hal, worin n und Hal die vorstehende Bedeutung besitzen, oder eine Gruppe der Formel

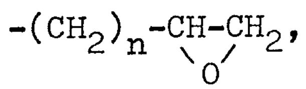

wie vorstehend definiert, wiedergibt, welches man mit einem Amin der Formel (V)

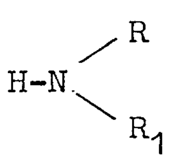

(V)

umsetzt, worin R und $R_1$ die angegebene Bedeutung besitzen, um zu einem Produkt der Formel ($I_A$)

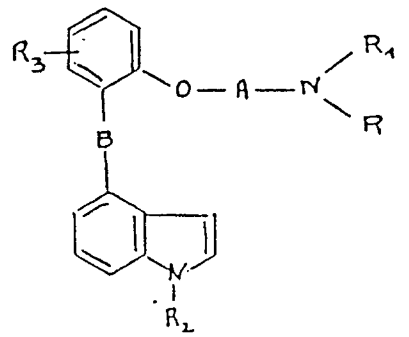

($I_A$)

zu gelangen, worin A, B, R, $R_1$, $R_2$ und $R_3$ die angegebene Bedeutung besitzen, welches man entweder isoliert und gewünschtenfalls in ein Salz überführt oder für den Fall, daß in dem Produkt der Formel ($I_A$) R und $R_1$ mit dem Stickstoffatom, an das sie gebunden sind, eine Gruppe

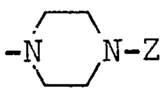

bilden, worin Z die vorstehend angegebene Bedeutung besitzt, man das entsprechende Produkt der Formel ($I_B$)

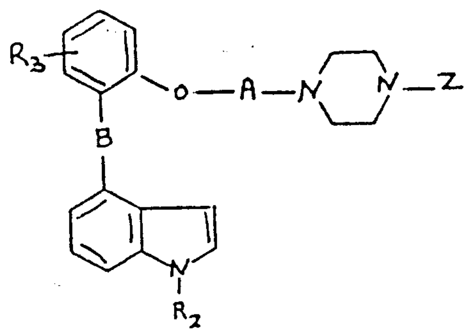

($I_B$)

worin A, B, $R_2$, $R_3$ und Z die vorstehend angegebenenBedeutungen besitzen, der Einwirkung eines Halogenierungsmittels unterzieht, um zu einem Produkt der Formel (VII)

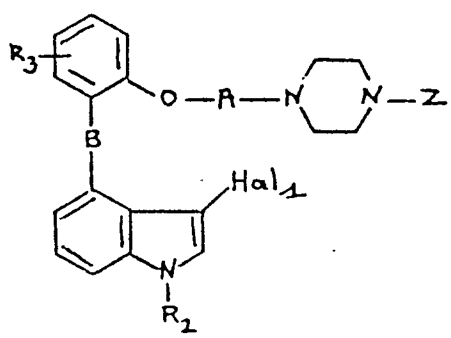

(VII)

zu gelangen, worin A, B, $R_2$, $R_3$ und Z die vorstehend angegebenen Bedeutungen besitzen und $Hal_1$ für ein Brom- oder Chloratom steht, welches man einer Hydrolyse unterzieht, um eine

Verbindung der Formel ($I_C$)

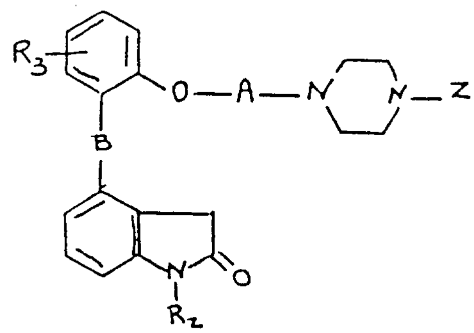

($I_C$)

zu erhalten, worin A, B, $R_2$, $R_3$ und Z die angegebenen Bedeutungen besitzen, welches man isoliert und gewünschtenfalls in ein Salz überführt,
oder man das Produkt der Formel (II) mit einem Derivat der Formel (VI)

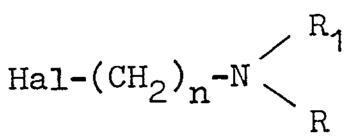

(VI)

umsetzt, worin Hal, n, R und $R_1$ die angegebene Bedeutung besitzen, um das Derivat der Formel ($I_A$) zu erhalten, worin A für eine Kette $-(CH_2)_n$ steht, welches man isoliert und gewünschtenfalls in ein Salz überführt, oder für den Fall, daß in dem Produkt der Formel ($I_A$) R und $R_1$ mit dem Stickstoffatom, an das sie gebunden sind, eine Gruppe

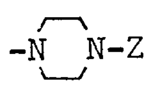

bilden, worin Z die angegebene Bedeutung besitzt, man das entsprechende Produkt der Formel ($I_B$) einem Halogenierungsmittel, dann einem Hydrolysereagens unterzieht, um das Produkt der Formel ($I_C$) zu erhalten, welches man isoliert und gewünschtenfalls in ein Salz überführt.

**2.** Verfahren gemäß Anspruch 1, dadurch gekennzeichnet, daß man von einem Produkt der Formel (II), worin $R_3$ ein Wasserstoffatom bedeutet, $R_2$ ein Wasserstoffatom oder einen Methylrest bedeutet, welches man mit einem Halogenid der Formel (III) Hal-G umsetzt, worin G eine Gruppe $-(CH_2)_nD$, wobei n für 2 oder 3 steht, oder eine Gruppe

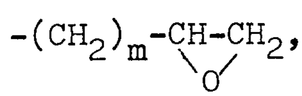

worin m für 1 steht, wiedergibt, und einem Produkt der Formel (V) oder (VI), worin $R_1$ und R mit dem Stickstoffatom, an das sie gebunden sind, eine Gruppe

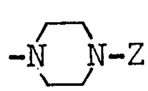

bilden, worin Z die in Anspruch 1 angegebene Bedeutung besitzt, ausgeht.

**3.** Verfahren gemäß Anspruch 1 oder 2, dadurch gekennzeichnet, daß man ein Produkt der Formel (I), worin die Substituenten $R_1$ und R mit dem Stickstoffatom, an das sie gebunden sind, eine Gruppe

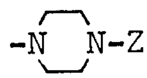

bilden, worin Z die vorstehende Bedeutung besitzt, A eine Kette

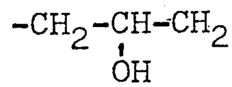

oder $-(CH_2)_3-$ oder $-(CH_2)_4-$ bedeutet, $R_3$ ein Wasserstoffatom wiedergibt, $R_2$ für ein Wasserstoffatom oder eine Methylgruppe steht, b für ein Wasserstoffatom steht und a und c gemeinsam eine zweite Bindung zwischen den sie tragenden Kohlenstoffen bilden, sowie deren Additionssalze mit Mineral- oder organischen Säuren herstellt.

**4.** Verfahren gemaß Anspruch 1, dadurch gekennzeichnet, daß man das 2-[3-[(1,1-Dimethylpropyl)-amino]-2-hydroxypropoxy]-N-(1H-indol-4-yl)-benzamid und dessen Benzoat und dessen neutrales Oxalat herstellt.

**5.** Verfahren gemäß Anspruch 1, dadurch gekennzeichnet, daß man das 2-[3-[(1,1-Dimethylethyl)-amino]-2-hydroxypropoxy]-N-(1-methyl-1H-indol-4-yl)-benzamid und dessen Hydrochlorid herstellt.

**6.** Verfahren gemäß Anspruch 1, dadurch gekennzeichnet, daß man das 2-[2-Hydroxy-3-[(1,1,3,3-tetramethylbutylamino]-propoxy]-N-(1H-indol-4-yl)-benzamid herstellt.

**7.** Verfahren gemäß Anspruch 1, dadurch gekennzeichnet, daß man das 2-[2-Hydroxy-3-[4-(diphenylmethyl)-1-piperazinyl]-propoxy]-N-(1H-indol-4-yl)-benzamid und dessen neutrales Oxalat herstellt.

**8.** Als neue industrielle Produkte die Verbindungen der Formel (VII), wie in Anspruch 1 definiert.

**9.** Verfahren zur Herstellung von pharmazeutischen Zusammensetzungen, dadurch gekennzeichnet, daß man als Wirkstoff zumindest eines der Produkte der Formel (I), wie in Anspruch 1 definiert, oder zumindest eines ihrer Additionssalze mit pharmazeutisch verträglichen Säuren in eine für diese Verwendung bestimmte Form überführt.

**10.** Verfahren zur Herstellung von pharmazeutischen Zusammensetzungen, dadurch gekennzeichnet, daß man als Wirkstoff zumindest eines der Produkte der Formel (I), wie in Anspruch 2 oder 3 definiert, oder zumindest eines ihrer Additionssalze mit pharmazeutisch verträglichen Säuren in eine für diese Verwendung bestimmte Form überführt.

**11.** Verfahren gemäß Anspruch 4, dadurch gekennzeichnet, daß man als Wirkstoff das 2-[3-[(1,1-Dimethylethyl)-amino]-2-hydroxypropoxy]-N-(1H-indol-4-yl)-benzamid und dessen Benzoat und dessen neutrales Oxalat verwendet.

**12.** Verfahren gemäß Anspruch 2 oder 3, dadurch gekennzeichnet, daß man als Wirkstoff das 2-[3-[(1,1-Dimethylethyl)-amino]-2-hydroxypropoxy]-N-(1-methyl-1H-indol-4-yl)-benzamid und dessen Hydrochlorid verwendet.

**13.** Verfahren gemäß Anspruch 2 oder 3, dadurch gekennzeichnet, daß man als Wirkstoff das 2-[2-Hydroxy-3-[(1,1, 3,3-tetramethylbutylamino]-propoxy]-N-(1H-indol-4-yl)-benzamid verwendet.

**14.** Verfahren gemäß Anspruch 2 oder 3, dadurch gekennzeichnet, daß man als Wirkstoff das 2-[2-Hydroxy-3-[4-(diphenylmethyl)-1-piperazinyl]-propoxy]-N-(1H-indol-4-yl)-benzamid und sein neutrales Oxalat verwendet.